# EUROPEAN PATENT APPLICATION

(11) **EP 3 664 095 A1**
(43) Date of publication of application: **10.06.2020**
(21) Application number: 18210751.6
(22) Date of filing: 06.12.2018
(51) Int. Cl.: G16B 15/30, G16B 15/20

(54) **METHOD AND COMPUTER PROGRAM FOR DETERMINING OR ALTERING A COFACTOR SPECIFICITY OF A TARGET ENZYME**

(71) Applicant: Universidade Nova de Lisboa, 2780-157 Oeiras (PT); Universidade do Minho, 4704-553 Braga (PT)
(72) Inventor: Resende, Tiago Filipe Coelho, 4760-860 Vila Nova de Famalicão (PT); de Almeida Pereira da Rocha, Isabel Cristina, 4710-864 Braga (PT); Soares, Cláudio Manuel Simões Loureiro Nunes, 2775-800 Carcavelos (PT)
(74) Representative: Schulz Junghans Patentanwälte PartGmbB

(57) **Abstract**

The invention relates to a method and a computer program for determining a cofactor specificity of a target enzyme, wherein the target enzyme is expected to use one of a first cofactor and a second cofactor based on an amino-acid sequence of the target enzyme, and/or for determining an amino-acid sequence of a target enzyme variant, wherein the variant is characterized by a cofactor specificity differing from that of the target enzyme, the method comprising at least the steps of: providing an atomic structure for each of both cofactors, wherein each atomic structure comprises cofactor atoms, and wherein cofactor atoms in the atomic structures that are located at the same corresponding locations in both atomic structures are selected; providing the amino-acid sequence of the target enzyme; determining an estimated target enzyme-cofactor structure comprising information on a spatial structure of the target enzyme bound to one of the cofactors; generating an interaction matrix for the target enzyme-cofactor structure, wherein the interaction matrix comprises entries relating the selected cofactor atoms to surrounding amino-acid residues of the target enzyme; determining a cofactor specificity of the target enzyme by providing the interaction matrix, particularly the entries of the interaction matrix, to a trained classifier that is configured to classify the cofactor specificity of the target enzyme based on the provided interaction matrix to either the first or the second cofactor.

## Description

The invention relates to a method and a computer program for determining a cofactor specificity of a target enzyme, wherein the target enzyme is expected to use one of a first cofactor and a second cofactor based on an amino-acid sequence of the target enzyme, and/or for determining an amino-acid sequence of a target enzyme variant, wherein the variant is characterized by a cofactor specificity differing from that of the target enzyme.

Systems biology foundations broadly rely upon well performed gene annotations. In this post-genomic era, given the large and exponentially growing amount of sequences being characterized, experimental determination of a protein's function is becoming unfeasible, due to its cost and time consumption. Current methodologies are based on pairwise sequence alignment and search for sequence homology to perform protein function annotation. However, the usage of such approaches in annotation pipelines tend to continuously propagate annotation errors across all sequenced organisms due to the attribution of outdated and unspecific functions to new annotated genes, impairing the discovery of new gene functions. Despite their usefulness and relevance, such methodologies fail in capturing essential information hidden in dissimilar areas of different sequences, such as cofactor specificity, which gravely impairs the understanding of an organism's metabolism.

Cofactors act in enzymatic reactions as redox carries and are important mediators for energy transfer in the cell. The lack of accuracy in determining cofactor usage in numerous genes severely affects, for example, genome-scale metabolic model reconstruction, as well as metabolic engineering and strain design endeavours, due to the potential identification of misleading reactions.

Nicotinamide adenine dinucleotide (NAD(H)) and nicotinamide adenine dinucleotide phosphate (NADP(H)), are the most wildly used cofactors in cell metabolism. These structurally similar molecules act as functional group transfer agents, being therefore consumed at the same rate of substrate consumption. Moreover, the uncertainty of their usage in metabolic reactions has a major impact in metabolic engineering applications, affecting both predictions and strain design results. When correctly characterized, enzyme modification by thorough structure redesign for cofactor specificity change can be undertaken, enabling the efficient processing of multiple desired biocatalytic transformations.

NAD(H) and NADP(H) are functionally equivalent cofactors used for storage and exchange of electrons in catalytic reactions. These cofactors are used by the majority of oxidoreductases, the largest class in Enzyme Commission. The only difference between these two molecules is a phosphate group in the adenine moiety, located on the opposite side from the chemically active nicotinamide moiety. Despite their apparent similarity, enzymes that use these cofactors tend to be specific for only one of them, enabling pathway regulation and chemical driving force maintenance by the cells, through heavy regulation of the levels of oxidized and reduced metabolic pools of NAD(P)(H).

Despite the efforts in identifying the cofactor specificity, very few studies go beyond pinpointing the specific residues Arginine and Aspartate and only for the phosphate moiety area. The best example is a study performed using ketol-acid reductoisomerases that showed that the presence of acidic residues at conserved phosphate binding positions are potential candidates of enzymes preferring NAD(H). Another problem is the fact that most studies are based on data often composed by small datasets or specific enzyme sub-classes, which can bias the results due to their sequence similarity, and are regularly characterized using visual interpretation or selection of positive cofactor change mutations.

Moreover, one of the most relevant even greater challenge is not only to determine the cofactor specificity of an unknown enzyme, but also to alter the cofactor specificity of enzyme and thus opening the gate for designing biosynthetic pathways, such as the case of nicotinamide adenine dinucleotide (NAD(H)) and nicotinamide adenine dinucleotide phosphate (NADP(H)) pathways.

Due to the structural similarity between NAD(H) and NADP(H), with their only difference residing in the presence of a phosphate group in the vicinity of the 2' hydroxyl of the adenosine ribose in NADP(H), specificity mechanisms are difficult to characterize, hindering rational approaches for performing NAD(P)(H) cofactor specificity reversal or specificity switching.

An object of the present invention is to provide a method and a computer program for determining a cofactor specificity of a target enzyme, wherein the target enzyme is expected to use one of a first cofactor and a second cofactor based on an amino-acid sequence of the target enzyme, particularly wherein the amino-acid sequence is devoid of 2D- or 3D-structural enzyme information. Another aspect of the invention is to provide a method and computer program for determining an amino-acid sequence of a target enzyme variant, wherein the enzyme variant is characterized by a cofactor specificity differing from that of the target enzyme, wherein the cofactor specificity of the enzyme variant is particularly switched from the first cofactor to the second cofactor. These objects are achieved by the device having the features of claim 1.

Advantageous embodiments are described in the subclaims.

According to claim 1 the method comprising at least the steps of:
i) providing particularly an information on an atomic structure for each of both cofactors, wherein each atomic structure comprises cofactor atoms, and wherein cofactor atoms in the atomic structures that are located at the same corresponding locations in both atomic structures are selected,
ii) providing the amino-acid sequence of the target enzyme,
iii) determining an estimated target enzyme-cofactor structure comprising information on a spatial structure of the target enzyme bound to one of the cofactors,
iv) generating an interaction matrix for the target enzyme-cofactor structure, wherein the interaction matrix comprises entries relating the selected cofactor atoms to surrounding amino-acid residues of the target enzyme, particularly wherein for each selected cofactor atom, entries are generated in the interaction matrix that comprise the counts of each amino-acid residue of the target enzyme within a predefined distance to the selected cofactor atom,
v) determining, particularly by means of a higher probability score, a cofactor specificity of the target enzyme by providing the interaction matrix, particularly the entries of the interaction matrix, to a trained classifier, particularly a trained support vector machine that is configured to classify the cofactor specificity of the target enzyme based on the provided interaction matrix to either the first or the second cofactor.

It is noted that the sequence of steps i) to v) and also sequence steps of the subclaims do not have to be carried out according to their numbering or listed sequence. Moreover, separate sequence steps can be executed simultaneously or be comprised in a single step or action. Particularly steps comprising the provision of features can be merged and be reduced to practice by for example providing the respective features mixed or in combination.

Ad item i) The provision of the atomic structure particularly comprises the retrieval of an information about the atomic structure of the cofactor. The term "atomic structure of the cofactor" particularly refers to the geometrical and spatial relative position of atoms in the cofactor to each other.

The first and the second cofactor particularly differ in their atomic structure in at least one atom, i.e. the atomic structure of one of the cofactors has at least one atom that is not present in the other cofactor. This can be by addition or replacement.

The selected cofactor atoms particularly reflect essentially the atoms common in both cofactors. Atoms in excess are not selected.

Therefore, independent of the cofactor the number of selected atoms is the same, even though the cofactors might not have the same number of atoms. In other words, the selected atoms form a common cofactor devoid of structural features characteristic for the first or the second cofactor.

The atomic structure (and more specifically the information on the atomic structure), can be provided or retrieved for example by a particularly electronic database and stored in a data storage for further processing

The atomic structure can be provided in a common data format for storing and readout the atomic structures. Moreover, the atomic structure can be provided in combination with other information such as for example in combination with the target enzyme-cofactor structure. It is explicitly noted, that it is not necessary to provide the atomic structure in a separate processing step. It is within the scope of claim 1 that the atomic structure is provided by any means.

Ad step ii) the provision of the amino-acid sequence of the target enzyme particularly refers to the provision or retrieval of information on the amino acid sequence of the target enzyme. In analogy to step i) the information can be stored in an electronic data format and processed accordingly.

The amino acid sequence can provide a numbering of the amino acids such that the amino-acid sequence can be determined by providing number in combination with the kind code of the amino acid. Each amino acid therefore can be associated with a number indicating its unique position in the sequence.

Ad step iii) determining an estimated target enzyme-cofactor structure comprising information on a spatial structure of the target enzyme to one of the cofactors.

The term "bound" in the context of the enzyme and the cofactor particularly also refers to a state of interaction of the enzyme with the cofactor, when the enzyme uses said cofactor, and thus a generic interaction configuration is adopted by the enzyme and the cofactor. Therefore, the term "bound" is to be understood in a broad sense throughout the specification.

The target enzyme-cofactor structure in analogy to the atomic structure of the cofactor comprises the relative positions and particularly orientations of the atoms of the cofactor as well as the amino acids of the target enzyme.

The target enzyme-cofactor structure can be provided, determined or stored in an appropriate data format.

It is noted that the target enzyme-cofactor structure particularly provides information about the positions of the cofactor atoms, including the selected cofactor atoms, with respect to the amino acid residues of the enzyme. Therefore, it is possible to determine distances between the cofactor atoms and amino-acids and other spatial relations between the cofactor and the amino acid residues of the enzyme.

The term "amino acid residue" particularly refers to an amino acid comprised by the enzyme and can particularly addressed by means of an index in the amino acid sequence of the enzyme.

While on some cases the target enzyme-cofactor structure might be known or otherwise inferred, in other cases the target enzyme-cofactor structure has to be determined, for example by a homology search method. The latter is typically the case, when the cofactor specificity of the enzyme is unknown.

In case the cofactor specificity is to be altered or switched from the first cofactor to the second cofactor (or vice versa), the target enzyme-cofactor structure is often known regarding only one of the cofactors, e.g. the first cofactor. As in subsequent steps particularly only the selected cofactor atoms are of importance, knowing the target enzyme-cofactor structure for one cofactor only, e.g. the first cofactor is suffices for proceeding with the next method steps.

It is further noted that the atomic structure of the cofactor might be provided (cf. step i)) only or exclusively together with, e.g. bound to the target enzyme. The same holds true for the provision of the amino-acid sequence of the target enzyme (cf. step ii)). Ad step iv) generating an interaction matrix for and particularly from the target enzyme-cofactor structure, wherein the interaction matrix comprises entries relating the selected cofactor atoms to surrounding amino-acid residues of the target enzyme, particularly wherein for each selected cofactor atom of the selected cofactor atoms, entries are generated in the interaction matrix that comprise counts of each amino-acid residue or amino-acid residue type of the target enzyme within a predefined distance to the selected cofactor atom.

The interaction matrix is also referred to as the cofactor neighbor residue profile matrix or CNRPM. The CNRPM can be in generated in a form of a look-up table or any other format that is suited to relate selected cofactor atoms with the surrounding amino acid residues of the target enzyme. In the context of the current specification these formats are considered equivalent and within the meaning of the term "matrix", as they can serve the same purpose.

Entries in the CNRPM particularly consist of a single value that provides the information of the surrounding amino-acids.

According to one embodiment for each cofactor atom M entries are provided, wherein equals to the number of particularly naturally occurring amino acids, i.e. typically 20, or amino acids that are comprised in the enzyme.

Thus, for N selected cofactor atoms and M amino acids, a total of N x M entries are generated. Each entry relating the n^{th} selected cofactor atom with the m^{th} amino acid Said n^{th} x m^{th} entry comprises for example the number of Lysines with in the surrounding of m^{th} selected cofactor atom, which can be for example a carbon atom. It is noted that for the purpose of unambiguity, the cofactor atoms, particularly the selected cofactor atoms, can be associated with an index allowing an identification of the cofactor atom in the atomic structure of the cofactor.

The term "counts" particularly refers to the number of occurrences, e.g. the frequency, the specific amino acid is found in the surrounding.

The term "surrounding" particularly refers to an atomic scale surrounding, i.e. in the order of Angstroms.

As the number of selected cofactor atoms is independent of the first and second cofactor, the interaction matrix particularly does not comprise cofactor atoms solely present in one of the two cofactors.

Moreover, the number of entries and particularly the dimension of the interaction matrix is independent of the first or second cofactor and is the same for both cofactors.

This allows for a machine learning method, such as the classifier, particularly the support vector machine to process to be trained with the same number of features for the first and second cofactor.

According to one embodiment of the invention, the predefined distance corresponds to a radius of less than 12A, more particularly less than 6A. "A" stands for Angstrom. According to this embodiment all amino-acid residues that are within the predefined distance of at least one of the selected cofactor atoms are counted in the respective entry of the CNRPM.

In step v) a cofactor specificity of the target enzyme is determined by providing the interaction matrix, particularly the entries of the interaction matrix, to a trained support vector machine that is configured to classify the cofactor specificity of the target enzyme based on the provided interaction matrix to either the first or the second cofactor.

The classifier, particularly the support vector machine is particularly a machine learning method that during a supervised training phase identifies a hyperplane that separates features from each other such that a classifier is built.

The classifier is trained in a supervised manner with datasets of target enzymes having a known cofactor specificity, particularly a known target enzyme-cofactor structure, more particularly a known associated-interaction matrix, which can be determined from the target enzyme-cofactor structure.

The features for training and for evaluation provided to the classifier are particularly the entries of interaction matrix.

A suitable classifier in form of a support vector machine, can be based for example on the scikit-learn library for python [3].

After training the classifier, particularly the trained support vector machine is used for classifying interaction matrices associated or derived from to a target enzyme-cofactor structure. The classification is for example done by means of a higher probability score output by the classifier for one of the two cofactors.

It is noted that the interaction matrix does not comprise specific structural features of the cofactors as only the selected cofactor atoms are comprised in the interaction matrix.

Thus, even if the target enzyme-cofactor structure is specific to the first or second cofactor the interaction matrix does not comprise these structural differentiating features.

This allows using a molecular structure template of a known enzyme-cofactor structure and adapt said template to a target enzyme having unknown cofactor specificity or an unknown target enzyme-cofactor structure without introducing or at least with minimizing a bias in the interaction matrix that is used for classification. Thus, the support vector machine classifies the cofactor specificity of a given target enzyme by means of the interaction matrix associated to the target enzyme and the selected cofactor atoms.

Therefore, special attention should be paid at the generation step of the target-enzyme-cofactor structure in case it is unknown.

The method according to the invention therefore allows determining and particularly predicting the cofactor specificity of a target enzyme, wherein only the amino-acid sequence of the target enzyme and the atomic structure of the cofactor is provided. The target enzyme-cofactor structure can be determined by means of a homology search and/or a modelling method.

According to an embodiment of the invention, the trained classifier provides for each selected cofactor atom and for each cofactor and for each amino acid a feature weight indicative for a cofactor specificity strength of the amino acid.

The cofactor specificity strength and the feature weight is a statistically determined value derived from a training set for the classifier, particularly wherein a higher feature weight for an amino acid indicates that the said amino acid has a supposedly large influence on the cofactor specificity, wherein a lower feature weight indicates a small influence on cofactor specificity.

According to another embodiment of the invention, particularly if the target enzyme-cofactor structure is unknown, the following steps are executed for determining the estimated target enzyme-cofactor structure:
- performing a homology search with the amino-acid sequence of the target enzyme in a protein structure database comprising information on molecular structures of enzymes bound to the first or the second cofactor,
- assigning the molecular structure comprising the enzyme that exhibits a highest degree of homology to the amino-acid sequence of the target enzyme as a molecular structure template,
- from the molecular structure template determining the target enzyme-cofactor structure for the amino-acid sequence of the target enzyme bound to the cofactor particularly by aligning particularly modelling the amino-acid sequence of the target enzyme to the molecular structure template.

A homology search can be performed by using homology models. Such models are for example created using Modeller [7], where sequence similarity search for template selection is performed using the Smith-Waterman local alignment [8], [9] in a local database composed by structures from the Protein Data Bank (PDB) bound to one of the cofactors, such as for example NAD(P)(H). Structural similarity evidencing a suitable template is particularly assume when two enzymes share an amino-acid sequence identity above 25%, i.e. more than 25% of the amino-acid residues are identical.

The atomic structure of the cofactor is particularly allocated in the aligned amino-acid sequence by allowing Modeller to transfer these molecules from the molecular structure template to the modelled structure, which is considered the target enzyme-cofactor structure.

Thus, according to this embodiment the target enzyme-cofactor structure estimated from the molecular structure template might comprise the cofactor bound to the target enzyme for which the target enzyme might not be specific. This issue is particularly resolved by generating the interaction matrix from the selected cofactor atoms only and corrected by determining the cofactor specificity of the target enzyme with the classifier, particularly the support vector machine based on the interaction matrix.

According to another embodiment of the invention, the classifier, particularly the support vector machine is trained by
- providing particularly a protein structure database, the protein structure database comprising information on molecular structures of a plurality of enzymes, wherein each molecular structure represents an enzyme bound to the first or the second cofactor, particularly wherein each molecular structure is provided in the same format as the target enzyme-cofactor structure, particularly wherein the atomic structure of the bound cofactor in relation to the amino-acid residues of the respective enzyme is comprised by the molecular structure.
- for each molecular structure, generating the interaction matrix particularly according to step iv), wherein each interaction matrix is associated to the respective cofactor of the molecular structure,
- training the classifier, particularly the support vector machine with the interaction matrices so that the classifier, particularly the support vector machine is trained to classify the cofactor specificity for the first or the second cofactor of an enzyme based on the entries of the interaction matrix.

The molecular structures can be provided by the PDB.

Once the classifier is trained, the classifier particularly estimates a probability for the target enzyme to be specific for the first and/or second cofactor. If probability for the first and/or second cofactor is greater than a threshold value it is classified to be specific for this cofactor.

According to another embodiment of the invention, the trained classifier, particularly the support vector machine provides a cofactor specificity probability for the first and/or the second cofactor for classifying the cofactor specificity of the target enzyme, wherein if said cofactor specificity probability exceeds a predefined threshold value, the target enzyme is classified to be specific to the cofactor with the probability exceeding said threshold value.

The cofactor specificity probability particularly assumes values between 0 and 1, wherein the threshold value for the probability is particularly larger than 0.5, more particularly larger than 0.8, even more particularly larger than 0.9.

According to another embodiment of the invention, the amino-acid sequence of the target enzyme having the cofactor specificity switched from the first cofactor to the second cofactor is determined by the steps of:
a) particularly providing a target enzyme cofactor structure with a target enzyme being specific to the first cofactor,
b) prior to step v), replacing at least one amino-acid in the interaction matrix,
c) determining whether the cofactor specificity determined in step v) is switched from the first cofactor to the second cofactor,
d) particularly repeating the steps b) to c), particularly until the cofactor specificity determined in step v) is switched from the first cofactor to the second cofactor.

Step a) this step can be achieved already in step iii). This target enzyme-cofactor structure serves as the basis for modifications on the enzyme, wherein form the target enzyme-cofactor structure the interaction matrix is generated according to step iv). Once the interaction matrix is established, at least one amino-acid residue of the amino-acid sequence of the target enzyme is changed. This can be done directly in the interaction matrix according to step b) and translated to the amino-acid sequence of the target enzyme resulting in an amino-acid sequence for an enzyme variant.

It is noted that changing one amino-acid residue of the amino-acid sequence of the target enzyme might result in multiple changes in the interaction matrix, in case said amino-acid residue is comprised in multiple entries of the interaction matrix.

The altered interaction matrix is then provided to the trained classifier, such that a cofactor specificity of the enzyme variant is determined. In case the specificity remains unaltered another or more amino-acids can be changed until the cofactor specificity of the associated enzyme variant is witched form the first cofactor to the second cofactor.

This embodiment allows for determining an amino-acid sequence of an enzyme variant having a switched cofactor specificity with respect to the original target enzyme.

According to another embodiment of the invention, for each of the two cofactors and for each of the selected cofactor atoms and for each amino-acid, particularly when it is within the predefined distance of the cofactor atom, a feature weight indicative of a cofactor specificity strength is determined or provided, particularly wherein the classifier determines and/or provides the feature weights after being trained, particularly wherein said feature weights are provided in form of a computer readable look-up table.

The plurality of feature weights associated to the first or second cofactor and to the respective cofactor atom is also referred to as impact matrix in the current specification.

This embodiment allows identification of amino-acids for each selected cofactor atom that have a large influence on cofactor specificity. The identification is achieved by means of comparably (with respect to estimated feature weights of other amino acids) large feature weight.

It is noted that the feature weights are determined for amino acids in the surrounding, particularly within the predefined distance of the respective cofactor atom. The feature weights are particularly not connected to a specific enzyme or amino acid sequence but represent a generalized property of the cofactor specificity resulting from the training of the classifier, particularly the support vector machine.

The feature weight can particularly assume values between 0 and 1 or scaled to this range.

According to another embodiment of the invention, a maximum impact matrix is determined or provided relating for each cofactor, each selected cofactor atom to the amino-acid having the largest feature weight for the respective cofactor specificity, particularly wherein the maximum impact matrix is determined and/or provided by the trained classifier, particularly the trained support vector machine, particularly wherein the maximum impact matrix stored as a computer readable look-up table.

The maximum impact matrix is particularly generated after training of the classifier is completed, wherein the maximum impact matrix does not necessarily need to be generated each time the trained classifier determines the cofactor specificity of the target enzyme.

The maximum impact matrix can be sorted according to the feature weight, particularly such that the selected cofactor atoms associated with the amino acids with the largest feature weights are in the first rows or columns.

According to another embodiment of the invention, the amino-acid sequence of the target enzyme, i.e. the enzyme variant, having the cofactor specificity switched from the first cofactor to the second cofactor is determined by the steps of:
- before the interaction matrix is provided to the classifier, particularly the support vector machine in step v), replacing M amino-acid residues in the interaction matrix corresponding to the amino-acid residues of the target enzyme with the M largest feature weights for the first cofactor, with the corresponding M amino-acid residues for the same cofactor atoms having the largest feature weight for the second cofactor, wherein M is a natural number, particularly wherein the maximum impact matrix is used for looking up M amino acids for the first and second cofactor,
- particularly providing the such altered interaction matrix to the classifier, particularly the support vector machine as the interaction matrix in step v),
- after step v), determining whether the specificity of the enzyme variant associated to the interaction matrix is switched with respect to the target enzyme.

The M amino-acids having the M largest feature weights of the corresponding cofactor atom can be for example looked up from the maximum impact matrix.

Particularly from the maximum impact matrix the cofactors having the amino acids with the largest feature weight for the first cofactor are identified and the amino acids for the same cofactor atoms are identified (particularly from the maximum impact matrix) that have the highest feature weight for the cofactor specificity for the second cofactor.

Then, the M amino-acids with the M highest feature weights for the first cofactor are replaced with the amino acids (for the same cofactor atom) having the highest feature weight for the second cofactor.

This embodiment particularly uses the maximum impact matrix, however it is possible to do so also by selecting the respective amino acids from feature weights determined in the impact matrix.

M is particularly between 1 and 10.

This embodiment provides a deterministic approach for replacing the amino-acids.

According to another embodiment of the invention, starting from an initial value for M, particularly M =1, the amino acid residue having the M highest feature weights for the first cofactor are replaced by the corresponding amino acids for the second cofactor, wherein M is incremented by one or more in case the specificity is not switched, until the cofactor specificity of the target enzyme is switched or a predefined maximum value for M is reached.

As elaborated above, the switching can be established by the classifier that determines the cofactor specificity particularly based on a cofactor specificity probability.

According to another embodiment of the invention, the amino-acid sequence of the target enzyme having the cofactor specificity switched from the first cofactor to the second cofactor is determined by the steps of:
I) before the interaction matrix is provided to the classifier, particularly the support vector machine in step v), determining particularly using the interaction matrix of the target enzyme and the impact matrix or maximum impact matrix, N amino-acid residues of the target enzyme with the N largest feature weights for the first cofactor, wherein N is a natural number, particularly 10,
II) repeatedly performing a stochastic evolutionary method for replacing the N amino-acid residues with other amino-acid residues in the interaction matrix, and for each cycle of the stochastic evolutionary method and for each amino-acid sequence retrieved from the cycle determining the cofactor specificity and the cofactor specificity probability with step v) with the classifier,
III) after step v), selecting at least one target enzyme with a switched cofactor specificity.

With the selection of the N, particularly ten most suitable mutable residue positions for cofactor specificity reversal respectively switching, and given the possibility of each residue position being mutated by the remaining particularly 19 amino acid residues, it is computationally expensive to predict/determine the cofactor specificity of every mutant combination for each theoretically possible enzyme variant.

To overcome this issue, the stochastic evolutionary method particularly implements a stochastic evolutionary algorithm. These optimization algorithms perform an evolution of a population by mimicking biologic events such as natural selection, here for example the random replacement of one or more of the N amino acids.

According to another embodiment of the invention, the evolutionary algorithms used in the stochastic evolutionary method for efficiently predict the optimal set of mutations to reverse cofactor specificity are implemented using *inspyred* [16], an open source framework for creating biologically-inspired computational intelligence algorithms in Python.

According to another embodiment of the invention, the stochastic evolutionary method executes a predefined number of evolution cycles, particularly 100 evolution cycles.

According to another embodiment of the invention, a plurality of evolutionary algorithms, particularly thee or five evolutionary algorithms, are comprised in the stochastic evolutionary method, wherein a first evolutionary algorithm replaces only one of the amino acid residues of the N amino acid residues having the highest feature weight for the first cofactor during a cycle of the stochastic evolutionary method, wherein a second evolutionary algorithm replaces two of the amino acid residues of the N amino acid residues having the N highest feature weights for the first cofactor during a cycle of the stochastic evolutionary method, wherein a third evolutionary algorithm replaces three of the amino acid residues of the N amino acid residues having the N highest feature weight for the first cofactor during a cycle of the stochastic evolutionary method and so on for the rest of the remaining evolutionary algorithms of the plurality of evolutionary algorithms.

The amino acids are replaced in the interaction matrix by randomly chosen amino acid.

According to another embodiment an elitism value was set to 2, keeping the best 2 scoring enzyme variants for the next cycle, wherein the score is determined form the cofactor specificity probability.

According to another embodiment, the next best scoring 50% of all enzyme variants, particularly 50 enzyme variants, are recombined using mutation operators, with a crossover rate of 0.9 and a mutation rate of 0.1. The crossover operator particularly uses the parameters of two variant enzymes and combines them, generating two new enzyme variants, while the mutation operator substitutes one element of the enzyme variant by another, randomly generated, wherein the remaining lowest scoring 48% of enzyme variants are discarded and newly generated enzyme variants with random mutations in the available mutable positions are incorporated in the population. The stochastic evolutionary method is particularly terminated when the maximum number of cycles is reached.

According to another embodiment of the invention, in step II) selected amino sequences are provided to a next cycle of the stochastic evolutionary method, wherein the amino-acid sequences are selected based on the highest cofactor probability for the second cofactor.

This allows a convergence of the stochastic evolutionary method to a switched enzyme variant.

According to another embodiment of the invention, the first cofactor is selected from one of the redox pairs NAD / NADH and NADP / NADPH, and the second cofactor is the other of the redox pairs, or vice versa, and wherein the target enzyme or the enzyme variant is either specific to NAD(H) or NADP(H).

NADH is also referred to NAD(H), NADPH is also referred to as NADP(H) and the two cofactors are also referred to NAD(P)(H) in an indiscriminatory fashion.

According to another embodiment of the invention, the first cofactor is selected from one of the redox pairs FAD / FADH2 and NADP / NADPH, and the second cofactor is the other of the redox pairs, or vice versa. FAD is short form for a flavin adenine dinucleotide.

According to another embodiment of the invention, the first cofactor is selected from one of the redox pairs FAD / FADH2 and NAD / NADH, and the second cofactor is the other of the redox pairs, or vice versa.

According to another embodiment of the invention, the target enzyme and/or the enzyme variant is synthesized or the amino-acid sequence of the enzyme variant and/or the determined cofactor specificity of the target enzyme or the enzyme variant is stored in an electronic storage or provided to a user of the method.

The problem is furthermore solved by a computer program and/or a computer program product comprising particularly the computer program instructions which, when the program is executed by a computer, cause the computer to carry out the method according to the invention.

The computer can for example be general purpose computer.

In the following exemplary embodiments of the inventions are disclosed fore illustrative purposes.

### Figure description

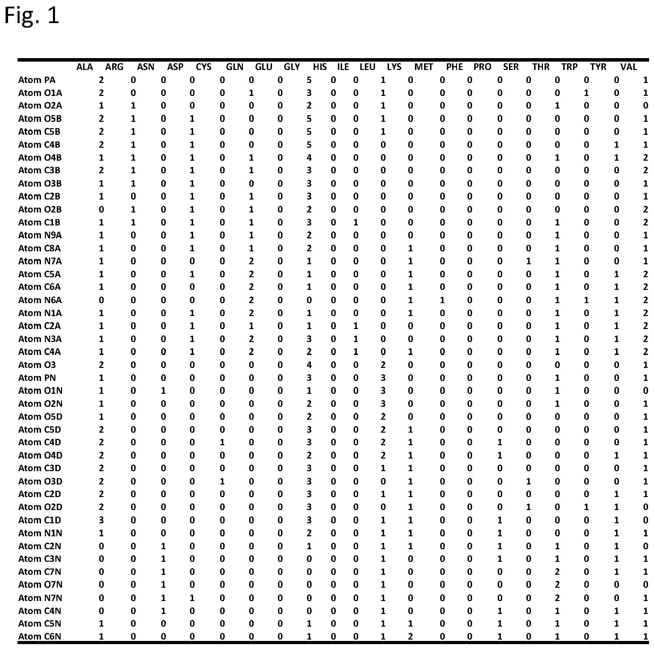

In Figure 1 a cofactor neighbor residue profile matrix generation is schematically depicted. Starting with the target enzyme-cofactor structure, the location of the cofactor as a reference is selected and the positions of the selected cofactor atoms are registered. For each selected cofactor atom the surrounding amino acid residues are selected, particularly those within 6 Å. The complete process is performed automatically. The end result encompasses a matrix of interactions between each cofactor atom and neighboring residues. The presented enzyme structure depicts a Dihydropteridine Reductase bound to NAD+ from Rat liver, with the EC 1.5.1.34. PDB id: 1DIR.

In the CNRPM the cofactor atoms are listed along the rows, wherein the amino acids are listed along the columns.

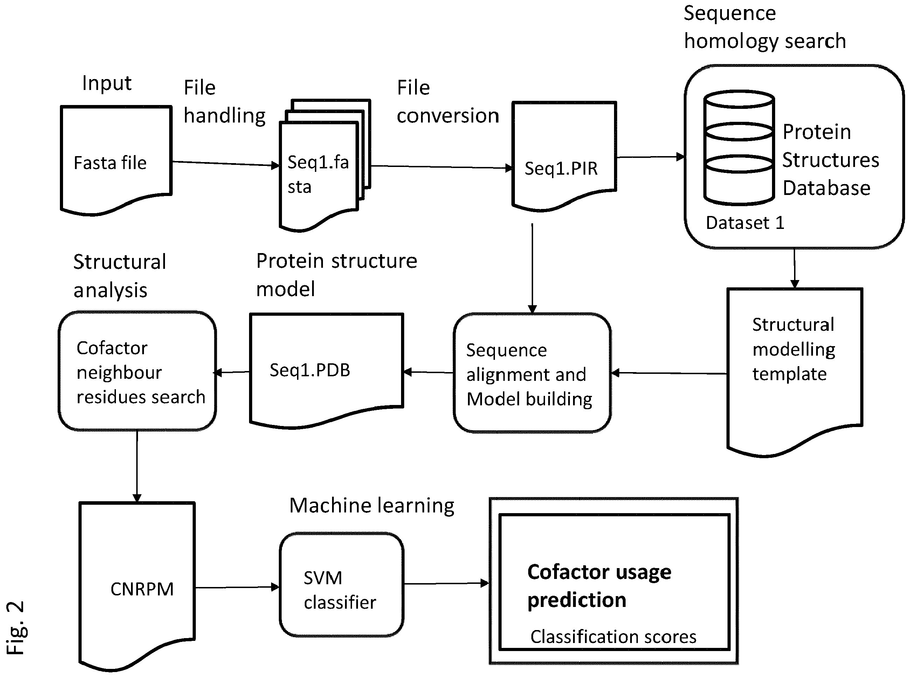

Figure 2 shows a flow diagram of an exemplary execution of the method according to the invention for target enzyme cofactor specificity prediction, also referred to as NiCofactor. Three input steps are performed upon provision of the amino acid sequence of the target enzyme that are related to file handling and data conversion to a suitable data format that is referred to as "Seq1.PIR" . In order to determine the estimated the target enzyme-cofactor structure, the sequence is used for a homology search for identifying a molecular structure that is close, i.e. whose enzyme has a most similar amino acid sequence, the molecular structure associated to the enzyme having the highest degree of homology is then selected and used as a molecular structure template.

In a next step, the target enzyme sequence is aligned (step "sequence alignment and Model building") with the molecular structure template in order to generate a presumable structure of the target enzyme bound to one of the cofactors.

The aligned sequence and the associated target enzyme-cofactor structure is then transferred in a PDB file format ("Seq1.PDB"). This format is particularly suitable and common for protein structures.

From the target enzyme-cofactor structure, the interaction matrix is determined by counting the amino acid residues of the target enzyme within a predefined distance, for example within 6A, of each selected co-factor atom (steps "Cofactor neighbour residues search" and "CNRPM"). The interaction matrix, particularly the entries of the interaction matrix, CNRPM, serve as features for the trained support vector machine and are therefore provided ot the SVM classifier. The support vector machine outputs a cofactor specificity probability for the target enzyme for being specific to one of the two cofactors. This is done n form of a classification score. If the score is above a predefined threshold value the target enzyme is considered to be specific for the respective cofactor.

Figure 3 shows schematically the process of identification of the amino acid residues of a target enzyme that have the highest features weights. With the CNRPM and the maximum impact matrix the ten amino acids having the highest feature weight are identified.

Some or all of these amino acid residues are replaced with another amino acid. Depending on the embodiment - a deterministic replacement method or a stochastic evolutionary method is applied.

In Figure 4 an embodiment of the stochastic evolutionary method is shown for replacing amino acids in the target enzyme to generate an enzyme variant with a switched cofactor specificity.

An initial population of altered enzymes is generated by randomly replacing at least one amino acid residues from the ten highest scoring amino acid residues of the target enzyme. Five evolutionary algorithms are used, wherein each evolutionary algorithms replaces a different number of amino acid residues, between one and five. Form this initial population comprising 100 altered enzymes, the cofactor specificity is determined according to the method of the invention in an "Evaluation step".

In this evaluation step, the two highest scoring altered enzymes are selected as elite candidates, the next 50 highest scoring altered enzymes are altered again by means of recombination provides. The score is particularly the cofactor specificity probability for the other cofactor. The remaining 48 altered enzyme having the 48 lowest scoring cofactor specificity probabilities for the other cofactor are discarded and new random mutations are introduced so that again 100 altered enzymes are provided for the next cycle. This process can be executed for each of the five evolutionary algorithms, such that for each number of replaceable amino-acids the best scoring altered enzyme can be identified. The stochastic evolutionary method is executed for 100 cycles. The best altered enzymes are the altered enzyme with the highest cofactor probability for the other, i.e. second cofactor (wherein the original target enzyme is specific to the first cofactor).

In the following various examples and exemplary embodiments for the reduction to practise are given.

### 1. Examples of cofactor specificity determination

### 1.1.1 Structure analysis and CNRPM generation

The generation of the cofactor neighbor residue profile matrix (CNRPM) for each NAD(P)(H) bound enzyme structure is built using the python computer programming language. Each target enzyme-cofactor structure is automatically handled and the distances between each selected cofactor atom and the amino acid residue neighbourhood (surrounding) are retrieved using the PDB module of the Biopython package [2].

Interactions between cofactor atom and neighbor residue are assembled in an interaction matrix and outputted, in order to be processed by the machine learning algorithm, particularly the classifier, more particularly the support vector machine.

### 1.1.2 CNRPM dataset extraction

All enzymes bound to one of the following ligand IDs: NAD/NAI/NAP/NDP, representatives of NAD⁺/NADH/NADP⁺/NADPH respectively, were sought after in the PDB and automatically retrieved and analyzed using the PDB module of the Biopython package.

Entries whose enzyme or cofactor structure were incomplete or disrupted were discarded. In order to overcome the problem of overfitting/biasing with structure duplicates or point mutations of the same enzymes with different entry codes, a redundancy threshold was set and applied to the sequences coding the retrieved enzyme structures. The selected threshold was set to 95% similarity in 90% of the sequence length, allowing the removal of duplicates and point mutations of the same enzymes.

### 1.1.3 Machine learning

Machine learning was used for solving the classification problem in the form of supervised learning. A support vector machine, the selected method, is applied using the scikit-learn library for python [3]. LIBSVM was the employed library and the radial basis function (RBF) was the chosen kernel function.

The developed CNRPM dataset was used as a training set and handled with the NumPy library for python [4]. Model performance was evaluated by measurements including accuracy, precision, Matthew's correlation coefficient (MCC) and area under curve of the receiver operating characteristics (AUC ROC). Accuracy refers to the closeness of a measured value to a standard or known value, precision refers to the closeness of two or more measurements to each other. MCC measures the prediction quality, taking into account over- and under- predictions and giving a complementary measure of the prediction performance [5]. MCC of 1 means a perfect prediction, and 0 denotes a completely random prediction. The receiver operating characteristic (ROC) curve [6], plots true positive rate on the y-axis against the false positive rate on the x-axis. The normalized area under curve of the receiver operating characteristics (AUC ROC) states a perfect prediction if the AUC value is 1, and a random guess if the value is 0.5.

### 1.1.4 Comparative modeling for structure analysis

Homology models are created using Modeller [7] and the modeller package for python, where sequence similarity search for template selection is performed using the Smith-Waterman local alignment [8], [9] in a local database composed by structures from PDB bound to one of the cofactors NAD(P)(H). Structural similarity evidencing a suitable template was assume when two proteins share a sequence identity above 25%

The structure of the Cofactor is correctly allocated in the modelled structures by allowing Modeller to transfer these molecules from the template to the modelled structure.

### 1.1.5 NiCofactor tool construction

The method for determining the cofactor specificity of an target enzyme is termed NiCofactor allowing high throughput NAD(P)(H) cofactor specificity prediction was built using the python programming language. For each sequence in the FASTA format used as input, the method initiates an individual project. The steps for generating CNRPMs and performing machine learning were also integrated in NiCofactor. Results are outputted by attributing to each analyzed sequence a cofactor specificity prediction and subsequent prediction score, indicative for the cofactor specificity probability.

### 1.1.6 NiCofactor result validation dataset

Curated information on cofactor, cofactor specificity, EC number, organism, sequence, literature and source information on enzymes using NAD(P)(H) were retrieved automatically from brenda-enzymes using SOAPpy, a tool for building SOAP clients and servers, implemented in python [10].

### 1.2 Results

### 1.2.1 Cofactor neighbor residue profile matrix (CNRPM) development

Characterizing structural information is a challenging task due to the overwhelming amount of information associated with the structure of a protein. The main focus was to retrieve all possible interactions between each cofactor atom and the nearest residues in the binding pocket of the target enzyme. With that in mind a tool that, given a characterized structure bound with NAD(P)(H) (in the PDB format), automatically returns a matrix of interactions between each cofactor atom and the surrounding amino-acid residues, at a distance of 6 Å. By ignoring the atoms related to the phosphate in the adenosine moiety of NADP(H), it is possible to create similar cofactor neighbor residue profile matrices (CNRPM) for both NAD(H) and NADP(H) cofactors, which is crucial to a well performing machine learning method, such as the support vector machine. Figure 1 depicts the cofactor neighbor residue profile matrix building process.

In these CNRPM, where each line refers to a selected cofactor atom (44 atoms) and each column refers to one of the twenty natural amino-acids, each value refers to the number of residues found. If, within the surroundings of an atom, a specific residue is not present, the value of that interaction is set to 0 (zero) in the interaction matrix. Thus, an interaction matrix with 20x44 entries is generated that encompasses 880 interaction values.

### 1.2.1.1 Building a comprehensive and representative CNRPM dataset

With the intent of applying the developed method in the construction of an accurate and representative dataset of CNRPMs, for unveiling the molecular determinants of cofactor specificity, a database of enzyme structures bound to NAD(P)(H) is assembled. To do so, (in January 13^{th} 2016) all enzyme structures bound to one of the cofactors NAD(P)(H) from the PDB were retrieved and analyzed. The total amount of structures collected was 2742, from which 148 were discarded due to incompleteness. With the removal of protein sequence redundancy, the final dataset encompassed 921 structures, being 491 structures bound to NAD(H) and 430 to NADP(H). Once the database was assembled and validated, the developed method was applied to all structures and a CNRPM was retrieved for every enzyme.

### 1.2.2 CNRPM dataset analysis and processing using Machine learning

Having built a large representative dataset of 921 CNRPMs, a support vector machine (SVM) algorithm was used to attribute cofactor preference/specificity based on the CNRPMs, while evaluating the performance of the method. The SVM training algorithm works by building a model, with categorized training examples, such as the CNRPMs (which are categorized as belonging to NAD(H) or NADP(H)), and representing them as points in a high-dimensional hyperplane, separated by category and divided by a clear gap between them. This allows the algorithm to assign a category to uncategorized new examples, based on the side of the hyperplane they fall. Performance is assessed by measuring how fine the division of categories is achieved [11].

By applying this algorithm to the CNRPMs dataset as a training set, an SVM model was created whose evaluation and performance parameters can be found in table 1.1. The created model achieved an accuracy of 96.2%, being able to correctly classify 886 CNRPMs as corresponding to NAD(H) or NADP(H) cofactors, with a precision of 96.03% and a Matthews correlation coefficient (MCC) of 0.92. The computed area under the receiver operating characteristic curve (AUC ROC) coefficient is 0.96. The confusion matrix displayed in table 3.1 evidences the high sensitivity and specificity of the model, with similar misclassification values in both NAD(H) and NADP(H) CNRPM.

Table 1.1 depicts the evaluation and performance parameters of the created SVM model. Accuracy, precision, MCC (Mathews correlation coefficient) and AUC ROC (area under the receiver operating characteristic curve) values (top) display the overall performance of the mode, indicating a well performing model. The Confusion matrix (bottom) evaluates sensitivity and specificity of the model.

**Table 1.1**

| | Accuracy | Precision | MCC | AUC ROC |
|---|---|---|---|---|
| SVM model | 96.20% | 96.03% | 0.92 | 0.96 |
| | | | | |

| Real cofactor | | | | |
|---|---|---|---|---|
| NAD(H) | NADP(H) | | | |
| 474 | 17 | NAD(H) | | Predicted cofactor |
| 18 | 412 | NADP(H) | | |

These results put in evidence that the type and number of residues present in the cofactor binding site have a crucial role in the specification of cofactor preference in the enzyme. Such results also demonstrate the possibility to predict/indicate cofactor preference in an enzyme by analyzing its cofactor neighbor residue profile using the method according to the invention.

### 1.2.2.1 SVM feature weights extraction and interpretation

The SVM model training works by attributing weights to features in the dataset (in this case a feature is a cofactor atom-residue interaction), allowing the correct separation of the instances in the hyperplane. Such separation is what enables the algorithm to classify a CNRPM as originated from an enzyme bound to NAD(H) or NADP(H). The extraction and interpretation of such metrics are of great importance in the identification of the crucial interactions between residue and cofactor atoms, and should allow to exactly pinpoint the set of relations in the CNRPM responsible for providing the cofactor preference to an enzyme. The extracted data, composed by 880 features and their respective weight in the SVM model, are presented in table A1 of the appendix. The highest extracted weight values correspond to 0.44991 for NADP(H) and 0.23609 for NAD(H). Despite the large amount of features, feature weight values from both NADP(H) and NAD(H) decrease rapidly from the heaviest values, leveling out in lighter features. This indicates that, despite the contribution of all features to the classification of the CNRPMs, some relations have a more significant role in classifying cofactor preference than others.

When analyzing the results, it was possible to observe that selected cofactor atoms from all parts of the cofactor structure contribute to specificity, despite the only difference between both cofactors being the presence of a phosphate molecule in the ribose from the adenine moiety. In fact, the fifteen heaviest features for both cofactors encompass atoms from adenine, ribose from adenosine, phosphates, ribose from nicotinamide ribose and nicotinamide.

Table 1.2 displays the fifty heaviest features for each cofactor along with the respective weight.

**Table 1.2 shows the SVM model feature weight distribution for NAD(H) (left) and NADP(H) (right). Feature weight is distributed in a decreasing order, starting from the heaviest. Columns depict the type of atom, amino acid (AA) and feature weight. Feature weights are divided into two sub columns for each cofactor.**

| NAD(H) | | | | | | NADP(H) | | | | | |
|---|---|---|---|---|---|---|---|---|---|---|---|
| Atom | AA | Weight | Atom | AA | Weight | Atom | AA | Weight | Atom | AA | Weight |
| C8A | ASP | 0.236 | C4B | ARG | 0.144 | O2B | ARG | 0.450 | C4N | LEU | 0.145 |
| O4B | SER | 0.214 | N3A | ASP | 0.143 | O2B | SER | 0.265 | C4A | ASN | 0.137 |
| C4B | ASP | 0.212 | O2B | LEU | 0.142 | O2B | LYS | 0.262 | N9A | TYR | 0.137 |
| C5B | ASP | 0.212 | O3B | GLU | 0.141 | C2B | ARG | 0.229 | O2D | ALA | 0.135 |
| O5B | ASP | 0.202 | N9A | LEU | 0.139 | 03 | GLY | 0.223 | O2A | ALA | 0.134 |
| C5A | LYS | 0.199 | C1B | ALA | 0.137 | O3B | ALA | 0.218 | O5B | SER | 0.133 |
| O2D | VAL | 0.193 | C4D | TYR | 0.135 | C4D | ASN | 0.217 | N1A | PRO | 0.132 |
| C2B | GLU | 0.179 | O1N | ARG | 0.134 | O1A | ALA | 0.186 | N7N | ASN | 0.129 |
| N6A | ALA | 0.179 | C3N | GLU | 0.128 | O2B | GLY | 0.180 | O1A | LYS | 0.127 |
| C5B | TRP | 0.177 | O5D | ALA | 0.128 | N1A | SER | 0.168 | N7N | ALA | 0.127 |
| O1N | LEU | 0.176 | N7N | ILE | 0.126 | C5B | LEU | 0.164 | O3B | LYS | 0.124 |
| C2B | GLY | 0.174 | N9A | GLU | 0.125 | C1B | ARG | 0.164 | C5D | ALA | 0.123 |
| N7A | PHE | 0.165 | O1A | ARG | 0.125 | O3D | TYR | 0.161 | C2B | SER | 0.123 |
| O2N | LEU | 0.162 | O3B | ILE | 0.125 | O7N | CYS | 0.159 | C4N | GLY | 0.119 |
| N7N | ASP | 0.157 | C8A | MET | 0.122 | PN | GLU | 0.159 | C8A | TYR | 0.117 |
| O2N | PHE | 0.157 | O5D | THR | 0.122 | C2N | ILE | 0.158 | 03 | ASN | 0.116 |
| O3B | PHE | 0.155 | O1N | ASN | 0.121 | C5A | TYR | 0.157 | O4D | THR | 0.115 |
| C5B | ALA | 0.153 | O7N | ARG | 0.120 | O2N | ASP | 0.153 | C4A | ALA | 0.114 |
| O3B | GLY | 0.152 | N3A | LYS | 0.120 | C3N | LYS | 0.150 | C4B | LEU | 0.114 |
| O2B | GLU | 0.152 | N1A | PHE | 0.120 | O5D | ILE | 0.149 | N1N | ASP | 0.114 |
| O1N | TYR | 0.152 | N9A | ILE | 0.120 | C4D | THR | 0.148 | C5B | CYS | 0.113 |
| N3A | TYR | 0.151 | C3B | ILE | 0.118 | N7A | TYR | 0.148 | O4B | GLY | 0.112 |
| N9A | ASP | 0.150 | C1B | SER | 0.118 | C2B | LYS | 0.147 | C2B | THR | 0.112 |
| O5D | GLN | 0.149 | C5A | PRO | 0.117 | O1A | SER | 0.146 | O7N | HIS | 0.111 |
| C2B | PRO | 0.148 | C3D | HIS | 0.117 | C6A | VAL | 0.145 | C8A | SER | 0.110 |

In Table 1.2, not only cofactor atoms from the entire cofactor structure are present, but also a large majority of the 20 natural amino acids residues are present in features from both cofactors. In the case of NAD(H), besides Aspartate, also Glutamate, Alanine, Leucine, Phenylalanine, Arginine and Isoleucine residues are frequently present in the displayed features, dispersed in interactions with atoms from the entire NAD(H) structure, being Cysteine the only amino acid residue not present in the first fifty features. In the case of NADP(H), again the most important interactions occur in atoms belonging to the adenosine moiety, with Arginine residues near the atom O2B being the heaviest feature, possibly due to the presence of the phosphate connected to that atom in NADP(H). Serine, Lysine, Glycine, Alanine, Asparagine and Tyrosine residues are the most frequent amino acid residues present in the first features, being absent from this group Tryptophan, Phenylalanine, Glutamine and Methionine residues.

### 1.2.3 NiCofactor cofactor specificity prediction method development

For prediction of target enzyme cofactor specificity for enzymes with an unknown target enzyme-cofactor structure, comparative modelling methods are used, as these methods not only allow processing newly sequenced enzymes or organisms, but also cope with the large existing gap between available sequences in Uniprot (93 million) and structures in PDB (almost 135 thousand, with only 42572 being directly linked to Uniprot as of October, 2017). To do so, a method was developed that implements functions for comparative modelling of protein structures using Modeller [7], a software that performs modelling by satisfaction of spatial restrains, through sequence alignment of the target sequence and known related structure templates. Through the integration of the developed methods with the resulting SVM model, a method was created that automatically performs cofactor preference prediction. With only the input of an amino acid sequence, a machine learning analysis of the modelled structural environment around the cofactor is performed. Figure 2 represents the pipeline developed within the built framework that enables the prediction of cofactor specificity. The developed framework is implemented in a computer program.

Figure 2 shows the developed framework pipeline. The displayed planes depict the sequence of events for cofactor specificity prediction. Starting from the left top, a Fasta file composed by the target enzyme amino acid sequence is provided to the method according to the invention, where they are structurally modelled, analyzed and classified.

### 1.2.3.1 Validation of NiCofactor cofactor specificity prediction tool using curated information

In order to validate the developed method and the machine learning model, a dataset with curated information on enzyme specificity was constructed. For that, Brenda-enzymes [1] was used and curated information on cofactor, cofactor specificity, EC number, organism, sequence, literature and source were retrieved. Firstly, the database was filtered for enzyme entries with NAD⁺, NADH, NADP⁺ or NADPH as cofactor, subsequently the cofactor commentary field was filtered for expressions indicating high cofactor specificity, such us, "absolute specificity", "specific", "totally specific", "dependent on", "strict", "no activity" or "required". This step enabled us to create a dataset of 404 distinct amino acid residue sequences of different enzymes with high cofactor specificity experimentally determined, originated from a combination of 198 EC numbers and 180 organisms. From the total amount of enzyme amino acid sequences, 189 are specific for NAD(H) and 215 for NADP(H). With the retrieved information present on the dataset, the amino acid sequences encompassed in the dataset were arranged and displayed in Fasta format, in a single Fasta file. This file was then uploaded and processed in the developed tool NiCofactor and predictions of NAD(P)(H) cofactor specificity were performed.

When analyzing the results obtained from the developed tool it was possible to observe that from the total 404 sequences analyzed, composing the dataset of curated information, the developed tool performed a cofactor prediction for 327 (81%) as around 11% (45) of the enzymes analyzed had their structure characterized and approximately 70% (282), despite not having their structure characterized, were found to have a suitable structural template, enabling structure inference by homology modeling. For 19% (77) of the enzymes analyzed, no structural template was found, impairing the possibility of a cofactor prediction. The overall accuracy in prediction of the method according to the invention was 83.5%. By further analyzing the machine learning model prediction output, it is possible to retrieve the predictions probability, which is an estimate from the model on how probable the prediction is correct. When plotting the prediction probability results we observed that the accuracy of the model tends to increase with the prediction probability, which opens the possibility of establishing a probability threshold that should improve the framework predictive capabilities.

The vast majority of predictions made by the SVM, have a very high probability score, with nearly 50% of the analyzed sequences having a cofactor prediction probability of at least 95%, according to the model. In fact, 73.4% (240) of the outputted predictions have a prediction probability above 80%. This results indicate that most of the prediction performed by the developed SVM model have a high probability of being correct. It is also possible to observe that the accuracy of the predictions made increases with the prediction probability score outputted by the model, which validates the prediction probability score. When the outputted prediction has a probability of at least 80%, the accuracy of the predictions increases to from 83.5% to 90%, and when the prediction probability surpasses 95%, model accuracy is 96%. The presented results validate the developed tool for performing NAD(P)(H) cofactor preference predictions on enzymes, using only the enzyme's amino acid residue sequence as input, which enables the prediction of cofactor preference in newly sequenced enzymes, or enzymes whose structures are yet to be characterized. To improve the prediction accuracy of the developed tool, a prediction probability threshold of 80% was set, which means that if the prediction probability of an analyzed sequence is, at least, 80%, the prediction is accepted as correct.

### 1.2.3.2 NiCofactor sensitivity analysis with case studies

In order to demonstrate the performance sensitivity of the method two experimentally characterized case studies encompassing homologue enzymes using distinct cofactors, were further analyzed. First, the case of *Azospirillum brasiliense'*s *α-*Ketoglutaric Semialdehyde Dehydrogenase Isozymes (KGSADH) is presented. According to Watanabe *et al.* [12] in *A. brasiliense,* KGSADH is involved in the conversion of α-ketoglutaric Semialdehyde to α-Ketoglutarate in an alternative pathway of L-arabinose metabolism. In his study it is described that this bacterium encodes for two different KGSADH isozymes, D-glucarate/D-galactarate-inducible KGSADH-II and hydroxy-L-proline-inducible KGSADH-III with significantly similar sequences. After physiological characterization, they revealed that KGSADH-II and KGSADH-III showed similar high substrate specificity for α-ketoglutaric semialdehyde and different cofactor specificity, being KGSADH-II, NAD⁺ dependent and KGSADH-III, NADP⁺ dependent. KGSADH-II and KGSADH-III have a sequence identity of 62.41%, with 332 identical residues in an alignment length of 532.

The second case presented regards to two alkyl alcohol dehydrogenase (ADH) genes from the long-chain alkane-degrading strain Geobacillus thermodenitrificans NG80-2 characterized by Liu, et al. [13]. Both ADH1 and ADH2 are able to oxidize a broad range of alkyl alcohols up to at least C30, as well as 1,3-propanediol and acetaldehyde, and share a sequence identity of 26%. For either enzyme, both NAD⁺ and NADP⁺ can be used as electron acceptor. However, NAD⁺ is the preferred cofactor for ADH1, while NADP⁺ is the preferred cofactor for ADH2.

With the presented information we went on to perform cofactor prediction and assess the capability of the developed tool to predict the cofactor preference of such similar enzymes. With none of the structures of the analyzed enzymes characterized, the framework applied structure modelling in order to perform a prediction. After processing all sequences using NiCofactor for performing cofactor predictions, the resulting output was analyzed. The method according to the invention classified the analyzed enzymes correctly, being KGSADH-II classified as NAD(H) binding with a prediction probability of 99.2% and KGSADH-III as NADP(H) with 64.7% probability, whereas for the ADH genes, ADH1 was classified as NAD(H) specific with a prediction probability of 80.6% and ADH2 predicted as NADP(H) specific with 95.7% of probability.

These results demonstrate the robustness of the method according to the invention, in correctly attributing NADP(P)(H) cofactor preference to enzymes, using the amino acid sequence of the target enzyme as input. The results from the performed predictions are displayed in table 1.3. In the case of KGSADH, possibly due to their similarity, the selected structure template for both enzymes was the same, 1EZ0.pdb, a NADP⁺ dependent Aldehyde dehydrogenase from *Vibrio harveyi,* characterized by Ahvazi *et al.* [14]. This enzyme has a sequence similarity of 48% with KGSADH-II and 47% with KGSADH-III, being its structure characterized with NADP⁺ in the binding pocket. Regardless of the type of bound cofactor in the template enzyme structure, the developed method was still able to correctly classify cofactor preference in the subject enzymes, being the prediction with higher probability from the opposite cofactor. ADH1 structure was modelled using an alcohol dehydrogenase structure from *Thermotoga maritima* (PDB: 1O2D), with a sequence identity of 37%, while ADH2 model template was a butanol dehydrogenase also from *Thermotoga* maritma (PDB: 1VLJ), with 48% sequence identity.

**Table 3.3 - Cofactor specificity prediction. KGSADH II and KGSADH III from Azospirillum brasiliense cofactor specificity prediction analysis show the predicted cofactor and associated probability. ADH1 and ADH2 from Geobacillus thermodenitrificans NG80-2 cofactor specificity prediction analysis show the predicted cofactor and associated probability. Template information is also displayed with PDB ID and crystalized cofactor, as well as subject and template amino acid sequence alignment identity percentage.**

| Fasta_ID | Predicted cofactor | Probability | Selected template | Alignment identity % |
|---|---|---|---|---|
| KGSADH-II | NAD(H) | 0.992 | 1 EZ0.PDB (NAP) | 48 |
| KGSADH-III | NADP(H) | 0.647 | 1 EZ0.PDB (NAP) | 47 |
| ADH1 | NAD(H) | 0.806 | 1O2D.PDB (NAP) | 37 |
| ADH2 | NADP(H) | 0.997 | 1VLJ.PDB (NAP) | 45 |

The fact that homologue enzymes are usually specific for only one of the cofactors impairs a deeper analysis of case studies with homologues that use different cofactors. Nonetheless, the studied cases still present a good indicator of the performance sensitivity achieved. These case studies also help demonstrating that enzymes within the same environment, and with very similar functions and sequences, do not necessarily use the same cofactors for catalysis, which is a common assumption when annotating enzymes using sequence homology information.

**Table A1 - The presented table encompasses all extracted feature weights from the developed support vector machine.**

| NADP | weight | NADP | weight | NADP | weight | NADP | weight |
|---|---|---|---|---|---|---|---|
| O2B_ARG | 0.449913142 | O3B_MET | 0.079733928 | O1N_LYS | 0.048374441 | PN_PHE | 0.020487316 |
| O2B_SER | 0.265379606 | C5A_LEU | 0.079072527 | C5D_ARG | 0.048085877 | PN_VAL | 0.020312879 |
| O2B_LYS | 0.261630434 | O5D_HIS | 0.078422943 | O2N_PRO | 0.048039874 | C6A_ARG | 0.020247682 |
| C2B_ARG | 0.228622965 | N3A_GLY | 0.078126706 | C1B_GLY | 0.047830205 | C5A_ASN | 0.020164465 |
| O3_GLY | 0.222471342 | C2D_ILE | 0.077926803 | O4D_PHE | 0.047278498 | C2A_PHE | 0.019981824 |
| O3B_ALA | 0.217947335 | O1A_ILE | 0.07767809 | O5B_GLY | 0.047202217 | C3D_THR | 0.019211148 |
| C4D_ASN | 0.21647244 | C3N_GLY | 0.077661844 | C2N_TRP | 0.046976252 | O3_PRO | 0.019170498 |
| O1A_ALA | 0.186032894 | PA_VAL | 0.076984447 | O3_TRP | 0.046775668 | C1D_TYR | 0.01907725 |
| O2B_GLY | 0.180156852 | O4B_TYR | 0.076807191 | C4A_GLN | 0.046168774 | O4D_CYS | 0.019057066 |
| N1A_SER | 0.167953722 | C8A_ALA | 0.076797825 | O2B_TYR | 0.046021503 | PN_GLN | 0.018273416 |
| C5B_LEU | 0.164328457 | C1D_ASN | 0.076774549 | O5B_ILE | 0.045863598 | O2A_TYR | 0.01808518 |
| C1B_ARG | 0.163757278 | C3N_SER | 0.076468785 | C1B_TYR | 0.045550284 | N1N_ARG | 0.017754525 |
| O3D_TYR | 0.161156286 | O3D_ASP | 0.076355444 | N1A_ALA | 0.045532792 | N3A_HIS | 0.017404048 |
| O7N_CYS | 0.15867393 | O1N_TRP | 0.07594696 | O2A_PHE | 0.04545846 | O5B_MET | 0.016805257 |
| PN_GLU | 0.158627537 | O3B_ASN | 0.07554993 | C3D_MET | 0.045343175 | O4D_MET | 0.016554396 |
| C2N_ILE | 0.157813268 | N3A_ASN | 0.07451086 | C2A_VAL | 0.044915605 | C2D_TYR | 0.016524546 |
| C5A_TYR | 0.157211897 | N7N_LYS | 0.074129083 | C4B_THR | 0.043327963 | O2D_GLY | 0.016109269 |
| O2N_ASP | 0.152875829 | C4D_LYS | 0.07406761 | C5N_HIS | 0.043243281 | O3_TYR | 0.015763729 |
| C3N_LYS | 0.149656203 | C4B_TYR | 0.073339554 | N7A_ALA | 0.04306512 | N3A_VAL | 0.01547717 |
| O5D_ILE | 0.14931893 | N6A_ILE | 0.073237285 | C1D_LYS | 0.042897475 | O4D_HIS | 0.015401676 |
| C4D_THR | 0.148308899 | C5D_PRO | 0.073147588 | O5D_TRP | 0.042875009 | C3B_PHE | 0.015107134 |
| N7A_TYR | 0.148010431 | C1B_THR | 0.072608212 | O3D_LEU | 0.042203353 | C4B_SER | 0.015050321 |
| C2B_LYS | 0.146967824 | O2D_PRO | 0.07220358 | N9A_GLY | 0.041931586 | O5D_TYR | 0.015017127 |
| O1A_SER | 0.145495323 | C2N_VAL | 0.0718949 | N1A_ILE | 0.04163029 | O1A_GLU | 0.01475175 |
| C6A_VAL | 0.145139302 | C6N_ASN | 0.071586964 | PA_ASN | 0.041202421 | C8A_GLY | 0.014641457 |
| C4N_LEU | 0.144604686 | O7N_MET | 0.071435417 | C5A_SER | 0.041119304 | C3B_LYS | 0.014594822 |
| C4A_ASN | 0.1366117 | C5D_ASN | 0.070929606 | O7N_SER | 0.040917906 | C4D_MET | 0.014577395 |
| N9A_TYR | 0.136561537 | C6A_GLY | 0.070840069 | C2A_MET | 0.040509358 | PN_PRO | 0.014217951 |
| O2D_ALA | 0.135126711 | C4D_ILE | 0.070215604 | C6A_TYR | 0.040119304 | C2A_CYS | 0.01413095 |
| O2A_ALA | 0.134424506 | O3_ALA | 0.069690483 | C5A_GLN | 0.039823784 | C5D_TRP | 0.014000081 |
| O5B_SER | 0.13329123 | O1N_HIS | 0.069401062 | PA_ALA | 0.039247128 | O2A_VAL | 0.013853835 |
| N1A_PRO | 0.131819349 | O3D_TRP | 0.068758458 | C6N_ARG | 0.03923571 | O2A_THR | 0.013831904 |
| N7N_ASN | 0.128466426 | N9A_THR | 0.068569727 | C5N_MET | 0.039171159 | O3D_ARG | 0.013697525 |
| O1A_LYS | 0.127108198 | C6N_ALA | 0.068517968 | N7A_ASN | 0.039122243 | O2D_PHE | 0.013391022 |
| N7N_ALA | 0.126482461 | C7N_GLN | 0.068492631 | C5B_ILE | 0.039102077 | C5B_TYR | 0.013353805 |
| O3B_LYS | 0.123755873 | PA_TYR | 0.067210455 | C6A_SER | 0.038986334 | C1D_TRP | 0.013208285 |
| C5D_ALA | 0.123037613 | O2A_LEU | 0.066556605 | O2B_GLN | 0.038486753 | O5B_PHE | 0.012902275 |
| C2B_SER | 0.122966829 | O4D_TYR | 0.06595204 | C5B_GLY | 0.038076815 | O3B_PRO | 0.012638936 |
| C4N_GLY | 0.11898915 | O4B_ARG | 0.065935256 | O2B_ILE | 0.037717059 | N7N_SER | 0.012619455 |
| C8A_TYR | 0.116865395 | C4N_ARG | 0.065840612 | O1A_CYS | 0.037609135 | C4N_ASP | 0.012426783 |
| O3_ASN | 0.115743489 | C4N_SER | 0.065697869 | O3D_LYS | 0.036670257 | C4N_MET | 0.012024085 |
| O4D_THR | 0.11469516 | O2N_GLN | 0.065514055 | C5D_TYR | 0.036588347 | O2B_TRP | 0.011954468 |
| C4A_ALA | 0.114322444 | C1D_GLU | 0.065489345 | C2N_LYS | 0.036131842 | C2D_SER | 0.011876911 |
| C4B_LEU | 0.113631168 | C4N_HIS | 0.065426674 | C2B_PHE | 0.035967943 | N3A_PRO | 0.011724681 |
| N1N_ASP | 0.113602654 | O3D_HIS | 0.064528665 | C5D_ILE | 0.035936608 | C1D_ARG | 0.011535813 |
| C5B_CYS | 0.113187311 | C5N_SER | 0.064480807 | O2D_ASP | 0.035202961 | C5B_PHE | 0.01140188 |
| O4B_GLY | 0.111937545 | C3D_PRO | 0.064476088 | O4B_VAL | 0.035068685 | O2A_HIS | 0.01126606 |
| C2B_THR | 0.111749487 | C1D_VAL | 0.064134033 | N7A_ASP | 0.034717747 | O5B_LYS | 0.011256246 |
| O7N_HIS | 0.110573667 | N1A_ASP | 0.064125337 | C5N_THR | 0.034520477 | C2A_LYS | 0.011124762 |
| C8A_SER | 0.110288278 | O7N_TYR | 0.064086413 | C3N_ASN | 0.034431891 | O2A_ARG | 0.01061547 |
| O5B_VAL | 0.109365284 | PN_ASP | 0.063669197 | C7N_LYS | 0.03412763 | O3B_TRP | 0.010596586 |
| O3_ASP | 0.108192747 | C2D_ALA | 0.063214388 | C2D_ARG | 0.034126311 | N1N_PHE | 0.00998952 |
| C1B_LYS | 0.108082034 | O3_MET | 0.062869632 | N1N_TYR | 0.033524849 | C7N_ALA | 0.00994264 |
| C4D_LEU | 0.107729603 | O1A_TRP | 0.062853765 | C5N_TRP | 0.033375128 | O2B_ASP | 0.009908968 |
| C1D_THR | 0.107220378 | C4D_ASP | 0.062836247 | C1D_SER | 0.032376306 | N7N_TRP | 0.009672741 |
| N7N_GLN | 0.107145492 | O7N_PHE | 0.06270243 | C5B_MET | 0.032376033 | N9A_SER | 0.009415228 |
| C2A_ALA | 0.105908542 | C5B_THR | 0.062417335 | C6A_GLN | 0.031523082 | N1A_LEU | 0.009285992 |
| C3N_THR | 0.105248594 | C6N_MET | 0.062197946 | C2N_ARG | 0.031276336 | O5B_PRO | 0.009048099 |
| C6A_ASN | 0.104591342 | PN_TRP | 0.061994385 | PN_HIS | 0.030665142 | C3D_ARG | 0.00856326 |
| C5N_ILE | 0.104010781 | C2D_VAL | 0.061925295 | O2N_GLY | 0.030550952 | N1A_CYS | 0.008427029 |
| N7N_PRO | 0.102646095 | PN_LYS | 0.061636747 | O7N_PRO | 0.030053576 | C6A_LEU | 0.008178712 |
| C2N_PHE | 0.10249854 | C3B_CYS | 0.061045003 | O4D_TRP | 0.029930128 | C4N_GLN | 0.008101571 |
| N3A_ARG | 0.101773524 | O1A_MET | 0.060950449 | C3B_GLY | 0.029781147 | C4A_VAL | 0.008086476 |
| C2A_THR | 0.101567253 | C8A_ARG | 0.060817792 | N1A_ARG | 0.029752999 | O5D_GLY | 0.007969447 |
| N6A_GLN | 0.101434154 | PA_CYS | 0.060276098 | C7N_GLY | 0.029649647 | C3B_ASN | 0.007766154 |
| N1N_GLN | 0.100911738 | C6N_TRP | 0.059803838 | O2B_HIS | 0.029521975 | C2B_TRP | 0.0072598 |
| C2D_GLU | 0.100833855 | O1A_ASN | 0.059279603 | C3N_ILE | 0.028964874 | N6A_ASN | 0.007186107 |
| C2N_ASN | 0.099646904 | PA_PHE | 0.058835203 | O2N_TRP | 0.028578168 | C2A_ARG | 0.006983245 |
| C2B_CYS | 0.098308531 | O5D_PRO | 0.057889556 | O7N_ASN | 0.028227782 | C1D_PHE | 0.006853563 |
| C2N_THR | 0.097889728 | C4N_PRO | 0.057582026 | N6A_LYS | 0.027873306 | N7N_LEU | 0.006283492 |
| N6A_ASP | 0.097880648 | O4B_CYS | 0.05742591 | O2N_LYS | 0.027710043 | C5D_GLN | 0.00605615 |
| C3D_TYR | 0.097789016 | C4D_SER | 0.057417109 | C3D_LYS | 0.027588124 | C3N_PRO | 0.005892243 |
| C3D_ASN | 0.096020327 | N9A_PRO | 0.056977578 | N1A_GLN | 0.027196561 | C7N_VAL | 0.005799317 |
| N7N_ARG | 0.095925003 | O4B_LEU | 0.056890567 | N6A_VAL | 0.027155871 | O1A_GLY | 0.00542776 |
| PA_GLU | 0.095654393 | C3N_VAL | 0.056484245 | C2N_GLY | 0.027000196 | O4B_ASN | 0.005285506 |
| C2A_HIS | 0.094314915 | O7N_GLU | 0.055914303 | C7N_HIS | 0.026998926 | N1A_TRP | 0.004883473 |
| O4D_VAL | 0.093174086 | O5D_ARG | 0.055821931 | O7N_LYS | 0.026956111 | C4D_GLN | 0.004734092 |
| O3D_PRO | 0.092015331 | O3B_CYS | 0.054819911 | C3B_SER | 0.026905237 | N9A_ARG | 0.004626553 |
| O7N_VAL | 0.091513198 | C2B_TYR | 0.054126689 | O3D_ILE | 0.026902842 | N3A_GLN | 0.003542283 |
| C4N_CYS | 0.089453556 | C7N_MET | 0.053802096 | N1N_ALA | 0.02674033 | O3D_SER | 0.003501124 |
| C5N_ALA | 0.088879631 | C1B_ASN | 0.05365572 | PN_ARG | 0.026713488 | C3D_GLY | 0.003266954 |
| C1D_LEU | 0.088099191 | O3D_PHE | 0.053469827 | C3B_GLN | 0.02669302 | O5D_LYS | 0.003223613 |
| O4D_GLY | 0.086767653 | C3D_LEU | 0.053229595 | C5N_CYS | 0.026574053 | C5A_CYS | 0.003056985 |
| N7N_MET | 0.086633406 | N3A_MET | 0.053096854 | C4D_HIS | 0.02642775 | C6A_HIS | 0.003020458 |
| O2N_VAL | 0.085984478 | C7N_GLU | 0.05280589 | O2B_CYS | 0.025727259 | C5D_GLU | 0.00274718 |
| C4A_PRO | 0.085348112 | PA_LEU | 0.0527438 | C4B_CYS | 0.025414997 | O5B_ASN | 0.002626459 |
| O7N_ILE | 0.084973072 | O5B_ALA | 0.052622602 | O3_SER | 0.02531283 | O2N_MET | 0.00250402 |
| C6N_PHE | 0.084854801 | O3B_ARG | 0.052168269 | C4A_HIS | 0.025217149 | C2D_ASN | 0.002268232 |
| O2A_MET | 0.084659482 | O5D_LEU | 0.052144021 | O2D_GLN | 0.025168633 | O3_LYS | 0.002168882 |
| N7A_GLY | 0.084155597 | O3D_CYS | 0.051815901 | O1A_HIS | 0.024616979 | N1A_HIS | 0.001854708 |
| O2D_GLU | 0.084149479 | C4A_SER | 0.051093903 | C4D_PRO | 0.024612369 | O1A_PHE | 0.001652292 |
| C2A_ASN | 0.083940647 | PA_MET | 0.051091304 | C6N_HIS | 0.024028726 | N1N_THR | 0.001652148 |
| N7N_TYR | 0.083794294 | C3D_VAL | 0.05093086 | C6N_ASP | 0.023679437 | C2N_TYR | 0.001482017 |
| PA_ASP | 0.083171344 | N3A_PHE | 0.050731905 | N7N_PHE | 0.023380668 | O5B_CYS | 0.001317108 |
| C4D_CYS | 0.083049838 | O2D_TRP | 0.050600777 | O5B_GLU | 0.023362986 | C5B_SER | 0.001232059 |
| C1B_CYS | 0.082678811 | C2B_HIS | 0.050513385 | PN_MET | 0.023060629 | N9A_CYS | 0.001219865 |
| O2B_THR | 0.082490082 | C6N_GLN | 0.050409382 | O1N_GLN | 0.022780128 | C5B_GLN | 0.001179896 |
| C3N_LEU | 0.081724665 | N7A_SER | 0.049681009 | N6A_LEU | 0.022696971 | O2A_TRP | 0.000815129 |
| N1N_HIS | 0.081721236 | C5N_ARG | 0.04940221 | C2B_GLN | 0.022212051 | C5A_ILE | 0.000680242 |
| O3_VAL | 0.080908382 | C3B_LEU | 0.049214786 | C2N_SER | 0.021588548 | C5D_ASP | 0.000586944 |
| C3N_GLN | 0.080883263 | C1D_PRO | 0.049053943 | N1N_TRP | 0.021172989 | C6N_GLY | 0.000356168 |
| N1N_ILE | 0.080273148 | C4N_THR | 0.0488634 | C3B_ARG | 0.021026055 | PA_ILE | 0.0000763 |
| | | O1A_TYR | 0.048695887 | O2D_CYS | 0.0206318 | | |
| | | | | | | | |

| NAD | weight | NAD | weight | NAD | weight | NAD | weight |
|---|---|---|---|---|---|---|---|
| C8A_ASP | 0.236094258 | C4B_VAL | 0.084678643 | C2A_GLY | 0.04585673 | PA_GLN | 0.024167113 |
| O4B_SER | 0.214267146 | C4N_GLU | 0.084495222 | C8A_CYS | 0.045674108 | N9A_HIS | 0.023710343 |
| C4B_ASP | 0.211914031 | C1B_PHE | 0.082788143 | C3D_ALA | 0.045302776 | N7A_LEU | 0.023256866 |
| C5B_ASP | 0.211619835 | O2B_PHE | 0.082187984 | C3B_MET | 0.045277989 | C8A_ILE | 0.023221567 |
| O5B_ASP | 0.201497995 | O3_ILE | 0.081837268 | PN_CYS | 0.045070493 | C3B_TYR | 0.023129246 |
| C5A_LYS | 0.199153626 | O4D_GLU | 0.081724154 | C7N_TYR | 0.044696847 | C2B_VAL | 0.02300447 |
| O2D_VAL | 0.192728146 | C4A_LYS | 0.081269877 | C4D_ARG | 0.044656503 | O2B_VAL | 0.022694831 |
| C2B_GLU | 0.1793113 | C4D_GLY | 0.080939478 | C5A_TRP | 0.044048925 | C5N_TYR | 0.022381938 |
| N6A_ALA | 0.178674412 | C6A_GLU | 0.080923251 | N6A_TRP | 0.044048925 | C4N_TRP | 0.022043873 |
| C5B_TRP | 0.177316428 | C3N_TYR | 0.080480348 | C1D_ILE | 0.043144806 | O2N_ASN | 0.021764038 |
| O1N_LEU | 0.176228984 | C1D_CYS | 0.080016833 | O2D_HIS | 0.042941727 | C2A_SER | 0.021614551 |
| C2B_GLY | 0.174419789 | O4B_PHE | 0.079708441 | PA_ARG | 0.042894018 | N6A_GLY | 0.02146111 |
| N7A_PHE | 0.16528297 | O2D_SER | 0.07945322 | C5A_VAL | 0.042803352 | C3D_CYS | 0.02135515 |
| O2N_LEU | 0.161914369 | O1N_VAL | 0.078945222 | C2B_MET | 0.042765752 | O5D_GLU | 0.021243463 |
| N7N_ASP | 0.156998091 | C5D_SER | 0.078576218 | C3B_ASP | 0.042701922 | N7A_VAL | 0.021039061 |
| O2N_PHE | 0.156590241 | C6A_LYS | 0.077817193 | C4A_CYS | 0.042634678 | N1A_TYR | 0.020921701 |
| O3B_PHE | 0.155184371 | C8A_ASN | 0.077790099 | PN_ALA | 0.042627867 | O1N_THR | 0.020897222 |
| C5B_ALA | 0.152835734 | N3A_LEU | 0.077742066 | C5A_ASP | 0.042546411 | N6A_CYS | 0.020775768 |
| O3B_GLY | 0.151913164 | N6A_PRO | 0.077616001 | C2N_GLU | 0.042497144 | O3_GLU | 0.020196977 |
| O2B_GLU | 0.151898457 | C2N_ALA | 0.077249401 | O5B_GLN | 0.042340288 | C5N_LEU | 0.02009685 |
| O1N_TYR | 0.151450549 | C7N_CYS | 0.076845666 | C1D_ASP | 0.041495819 | C4B_GLN | 0.019926272 |
| N3A_TYR | 0.150626291 | O3D_GLY | 0.076306045 | N9A_TRP | 0.04128563 | C1B_GLN | 0.019633755 |
| N9A_ASP | 0.149611431 | C3D_GLN | 0.075904853 | C2A_TRP | 0.041271331 | N7N_GLY | 0.019561879 |
| O5D_GLN | 0.148609502 | C5D_CYS | 0.075202986 | N3A_TRP | 0.041271331 | C4B_ILE | 0.019405753 |
| C2B_PRO | 0.147532603 | C6N_GLU | 0.074741328 | C2B_ALA | 0.041255388 | C7N_ARG | 0.019372093 |
| C4B_ARG | 0.143967247 | C5A_THR | 0.074527957 | N1A_THR | 0.041026461 | 01A_ASP | 0.019244841 |
| N3A_ASP | 0.14300716 | C2N_ASP | 0.074459978 | C2N_LEU | 0.040888207 | 02DLYS | 0.019194013 |
| 02B_LEU | 0.141964964 | 02A_GLY | 0.073382866 | 05D_VAL | 0.040156633 | 01N_PHE | 0.019158342 |
| 03B_GLU | 0.141180163 | 04B_HIS | 0.073219477 | 03B_GLN | 0.039927468 | C5D_THR | 0.019113421 |
| N9A_LEU | 0.139362426 | C6N_LYS | 0.072117998 | 03D_ALA | 0.039896181 | C8A_GLN | 0.018997788 |
| C1B_ALA | 0.136457677 | C1B_TRP | 0.070639586 | N1N_GLY | 0.039653791 | C3N_ARG | 0.018983506 |
| C4D_TYR | 0.13469436 | C5A_ALA | 0.069382114 | 03B_HIS | 0.039458724 | 04D_SER | 0.018702347 |
| 01N_ARG | 0.133895846 | 02D_THR | 0.069185612 | C3D_ILE | 0.039401154 | C3N_ASP | 0.018453586 |
| C3N_GLU | 0.127710335 | C3B_VAL | 0.069108754 | N6A_ARG | 0.039362501 | C6A_CYS | 0.018355811 |
| 05D_ALA | 0.127675979 | C5N_ASN | 0.069004812 | 01N_CYS | 0.039281688 | 03D_GLN | 0.018256316 |
| N7N_ILE | 0.126107558 | C5N_GLY | 0.068747065 | N6A_SER | 0.03921673 | 02N_GLU | 0.01817712 |
| N9A_GLU | 0.125364201 | C2B_ILE | 0.0686374 | C2D_MET | 0.03895439 | C1D_GLN | 0.017972709 |
| O1A_ARG | 0.124914545 | O2D_LEU | 0.068182443 | C5D_VAL | 0.038878843 | C3B_HIS | 0.017919884 |
| O3B_ILE | 0.124643569 | C6N_LEU | 0.068135623 | C5D_LEU | 0.038872236 | C4N_PHE | 0.017696368 |
| C8A_MET | 0.122164263 | O1N_GLU | 0.068107524 | C1B_MET | 0.038286853 | C2D_TRP | 0.01744367 |
| O5D_THR | 0.122063447 | C5B_ARG | 0.068011606 | 04D_PRO | 0.038198556 | C2D_CYS | 0.016935276 |
| 01N_ASN | 0.121086105 | C4D_TRP | 0.067954707 | O1N_PRO | 0.03813499 | O4D_LYS | 0.016476418 |
| O7N_ARG | 0.119867381 | O4D_ASP | 0.066602311 | N1N_SER | 0.038079186 | C4B_TRP | 0.016383175 |
| N3A_LYS | 0.119833863 | C7N_THR | 0.0658177 | C6A_PHE | 0.038067227 | C7N_ASP | 0.016178585 |
| N1A_PHE | 0.119547183 | C3N_HIS | 0.065214102 | C5D_GLY | 0.038048449 | PA_LYS | 0.016159851 |
| N9A_ILE | 0.119507247 | C8A_THR | 0.065129865 | C5B_ASN | 0.038045075 | C2A_PRO | 0.016101081 |
| C3B_ILE | 0.117697785 | O7N_LEU | 0.064726578 | C6A_ASP | 0.037830366 | C5N_VAL | 0.016097103 |
| C1B_SER | 0.117485882 | C4D_PHE | 0.064478493 | N7A_LYS | 0.037771646 | N3A_SER | 0.015498109 |
| C5A_PRO | 0.116871565 | C1D_ALA | 0.064318425 | O1A_THR | 0.037593202 | C3D_SER | 0.015402666 |
| C3D_HIS | 0.116540224 | C5D_LYS | 0.063959291 | C4A_ARG | 0.037365628 | N1N_MET | 0.015128158 |
| C8A_HIS | 0.115036079 | C4N_LYS | 0.063207444 | C6A_TRP | 0.037202519 | C5B_PRO | 0.015098312 |
| C1B_GLU | 0.114836695 | 03D_MET | 0.062607471 | 07N_TRP | 0.037119054 | C2D_LEU | 0.014951664 |
| 04B_PRO | 0.114719939 | 05B_LEU | 0.061997954 | 03D_VAL | 0.036711513 | O2A_CYS | 0.014949863 |
| C5A_GLY | 0.114416309 | O2A_ASN | 0.061732721 | C8A_LEU | 0.036654066 | O3DGLU | 0.014843767 |
| C2A_ASP | 0.113940715 | N6A_THR | 0.061577713 | N1N_GLU | 0.036633975 | O2N_SER | 0.014469964 |
| C3N_ALA | 0.113621507 | C7N_ILE | 0.061382838 | C4A_ILE | 0.036210335 | O2B_MET | 0.014381374 |
| PN_ASN | 0.113168012 | O3_GLN | 0.061367368 | C4A_TRP | 0.036192205 | C3D_GLU | 0.013753582 |
| N6A_HIS | 0.113024505 | C2N_HIS | 0.061266738 | C2N_GLN | 0.035567033 | O2A_PRO | 0.013548182 |
| C5A_MET | 0.112329829 | C2N_PRO | 0.060988083 | C3B_TRP | 0.034880319 | N7A_GLN | 0.012765498 |
| PA_GLY | 0.11165329 | C6N_THR | 0.060959542 | 05B_HIS | 0.03477746 | C3D_TRP | 0.012640582 |
| O2A_GLU | 0.111244424 | PA_THR | 0.060328547 | C1D_MET | 0.0347337 | 01N_ALA | 0.012390965 |
| O4B_GLU | 0.109619937 | C6N_TYR | 0.060307835 | C5N_ASP | 0.034172545 | 04B_THR | 0.012108389 |
| C4D_ALA | 0.108202492 | N7N_VAL | 0.060165214 | 04B_ALA | 0.034119062 | 04D_GLN | 0.012038157 |
| O2B_ASN | 0.108147068 | 05D_CYS | 0.059410492 | C4A_GLY | 0.033689269 | N1N_ASN | 0.011838299 |
| O2D_ARG | 0.108038157 | C3N_PHE | 0.059088755 | O4D_ALA | 0.033586427 | N9A_LYS | 0.011775881 |
| C7N_ASN | 0.107213425 | 02N_CYS | 0.059056218 | 01N_GLY | 0.033556184 | C2D_GLY | 0.011721179 |
| O1N_ILE | 0.106640874 | N1A_ASN | 0.058117727 | C4B_GLY | 0.033344892 | O7N_GLN | 0.011160851 |
| C2A_TYR | 0.106180162 | PN_GLY | 0.057992715 | 03_PHE | 0.033049727 | 01N_MET | 0.011066817 |
| C8A_PHE | 0.105426384 | 05D_ASN | 0.057907553 | C3B_THR | 0.032956614 | C1B_HIS | 0.010953044 |
| N3A_THR | 0.104176435 | 02N_ARG | 0.057788184 | C3B_PRO | 0.032916451 | C3B_ALA | 0.010883357 |
| N1N_PRO | 0.102629164 | C4D_VAL | 0.057773452 | C5B_GLU | 0.032826056 | PN_TYR | 0.010703707 |
| OSD_SER | 0.10245746 | C2N_CYS | 0.057688219 | C7N_TRP | 0.032662711 | N9A_ALA | 0.010443668 |
| C4B_GLU | 0.102381935 | N9A_VAL | 0.057370037 | C5N_GLN | 0.032605112 | C4B_ASN | 0.010442276 |
| 07N_ALA | 0.102269399 | C1B_ILE | 0.05698411 | 04B_TRP | 0.032570682 | N9A_GLN | 0.009324513 |
| C5B_LYS | 0.102210074 | 02B_ALA | 0.056981668 | C4B_PHE | 0.032417942 | N7A_GLU | 0.00883231 |
| C2A_GLN | 0.101828872 | C4N_TYR | 0.056651445 | C5B_HIS | 0.03236729 | C5A_PHE | 0.008825354 |
| O1A_GLN | 0.101685897 | C3D_ASP | 0.056555527 | N1A_GLY | 0.03232162 | 03B_LEU | 0.008819554 |
| C8A_GLU | 0.100650034 | C6A_PRO | 0.056529942 | N3A_CYS | 0.032317815 | C6N_PRO | 0.008639054 |
| N7N_THR | 0.099880328 | C1D_HIS | 0.056345666 | C1B_PRO | 0.031301457 | N1N_LEU | 0.008632498 |
| O7N_THR | 0.099664754 | O3D_ASN | 0.056314177 | O2B_PRO | 0.03121384 | C5D_HIS | 0.007959814 |
| C4N_ALA | 0.098701899 | C4B_HIS | 0.056175761 | O4D_ASN | 0.030787145 | C2D_ASP | 0.00768146 |
| O3D_THR | 0.097248115 | C4A_ASP | 0.055724142 | N7A_TRP | 0.030680017 | O1A_LEU | 0.007670774 |
| 04B_ASP | 0.096754876 | C8A_TRP | 0.055617695 | PN_LEU | 0.029719512 | C5A_HIS | 0.007650503 |
| C8A_LYS | 0.095315866 | C5N_PRO | 0.055392821 | 02A_GLN | 0.029703758 | 03B_TYR | 0.007502293 |
| C1B_ASP | 0.094624659 | O3_CYS | 0.055226603 | 05D_MET | 0.029487202 | C2D_GLN | 0.007488351 |
| N6A_GLU | 0.094601341 | N6A_PHE | 0.054991735 | PA_TRP | 0.029056729 | C2A_LEU | 0.007471721 |
| N7N_CYS | 0.094435354 | C4A_GLU | 0.05479858 | 03_THR | 0.029037937 | O3_ARG | 0.006893838 |
| N3A_GLU | 0.094044915 | C2B_LEU | 0.053824457 | 02D_ILE | 0.028969805 | PN_THR | 0.006589932 |
| C2N_MET | 0.093635101 | 05B_ARG | 0.05370051 | C2D_PHE | 0.028951052 | C6N_ILE | 0.006317509 |
| C7N_SER | 0.093273592 | C3N_TRP | 0.053645898 | C2D_THR | 0.028863737 | O3B_SER | 0.00628109 |
| N7A_PRO | 0.092887422 | C5N_LYS | 0.053172349 | C6A_THR | 0.028566318 | N1N_CYS | 0.006183564 |
| C2A_GLU | 0.092512112 | O4B_GLN | 0.053113516 | C7N_PHE | 0.0284569 | O2N_HIS | 0.006143172 |
| C8A_VAL | 0.092039322 | N1A_VAL | 0.052949956 | C2D_HIS | 0.028138399 | C4B_ALA | 0.005844543 |
| C2D_PRO | 0.091974416 | N7A_ILE | 0.052668712 | O2A_SER | 0.027907848 | O2A_ASP | 0.005816768 |
| C5A_ARG | 0.091774884 | C2B_ASN | 0.052129331 | C8A_PRO | 0.027839496 | O2N_ILE | 0.00570703 |
| O7N_GLY | 0.091607528 | 02A_LYS | 0.052029338 | C2D_LYS | 0.027755749 | N3A_ILE | 0.005557604 |
| C4A_PHE | 0.091478957 | O5B_TRP | 0.051926206 | C5B_VAL | 0.027696387 | C4D_GLU | 0.005447283 |
| N7A_HIS | 0.091434955 | PN_ILE | 0.051876557 | C4N_VAL | 0.027305555 | O1N_ASP | 0.005178466 |
| C3B_GLU | 0.09139035 | C3N_CYS | 0.050868751 | O2N_ALA | 0.027160743 | O3B_VAL | 0.004899055 |
| C4A_LEU | 0.090872072 | C4B_MET | 0.050634 | C7N_LEU | 0.027019403 | C4N_ASN | 0.003532887 |
| O3B_ASP | 0.090720383 | C6A_ILE | 0.050325083 | O2D_ASN | 0.026645431 | C3D_PHE | 0.003504186 |
| C4B_PRO | 0.09067722 | O1N_SER | 0.049049516 | O3_LEU | 0.026576686 | C6N_VAL | 0.003094678 |
| N7A_ARG | 0.090528536 | O7N_ASP | 0.048624583 | O5D_PHE | 0.026565767 | O5B_TYR | 0.002555715 |
| N7A_MET | 0.090306467 | N6A_MET | 0.048577237 | PA_HIS | 0.026455381 | C3N_MET | 0.002362533 |
| C1B_LEU | 0.089465086 | 04D_ARG | 0.048423773 | O5B_THR | 0.026232191 | C5N_GLU | 0.002179866 |
| C5A_GLU | 0.088046164 | O2D_TYR | 0.048363667 | C6A_MET | 0.026165631 | O3B_THR | 0.001955582 |
| N7A_THR | 0.088005608 | PA_PRO | 0.048103131 | C4B_LYS | 0.026110914 | O1A_PRO | 0.001622565 |
| N7N_GLU | 0.087465764 | C6N_CYS | 0.047847513 | C5D_MET | 0.025805668 | C1D_GLY | 0.001541014 |
| N3A_ALA | 0.087417513 | C6N_SER | 0.047755285 | N1A_LYS | 0.025796286 | C4A_TYR | 0.001506384 |
| N9A_MET | 0.087399922 | 02D_MET | 0.047508299 | C5N_PHE | 0.025618651 | 04B_ILE | 0.000794275 |
| N9A ASN | 0.08732569 | 02A_ILE | 0.047073498 | N1A_MET | 0.025546119 | N7A_CYS | 0.00025527 |
| O2N_TYR | 0.087107984 | PA_SER | 0.046824837 | C4A_THR | 0.02553769 | O5D_ASP | 0.000240182 |
| N9A_PHE | 0.086900914 | N7N_HIS | 0.04665957 | N1A_GLU | 0.025466655 | O4D_LEU | 0.000196388 |
| O3_HIS | 0.08674575 | C1B_VAL | 0.04659479 | C2A_ILE | 0.025021287 | C5D_PHE | 0.000107303 |
| O4D_ILE | 0.086687568 | C7N_PRO | 0.04631959 | C4A_MET | 0.024798013 | PN_SER | 0.000103847 |
| O2N_THR | 0.086066019 | O4B_MET | 0.046008828 | C4N_ILE | 0.024773603 | O1A_VAL | 0.0000672 |
| C6A_ALA | 0.085894582 | N6A_TYR | 0.045938949 | O4B_LYS | 0.024320924 | N1N_VAL | 0.0000641 |
| C2B_ASP | 0.085308204 | | | | | | |

### 2.0 Examples of switching the cofactor specificity of an enzyme

Here, detailed embodiments of a method for the in silico efficient conversion of NAD(P)(H) cofactor specificity in structurally non-characterized enzymes are disclosed.

Firstly, a method for identifying and producing an ordered list containing the most influential residues for cofactor specificity in a given enzyme structure was developed, using the SVM predictive model and CNRPM (Cofactor Neighbor Residue Profile Matrix) as elaborated previously. The created list is assumed as containing the theoretical optimal set of residue positions suitable for point mutations conferring cofactor specificity reversal.

Secondly, two methods were developed in order to identify the optimal set of point mutations required for achieving cofactor specificity change. For that, two distinct approaches were implemented, being one deterministic, using the gathered information on the most influential residues for both cofactor specificities; and the other stochastic, using evolutionary algorithms to locate the optimal set of mutations capable of reverting cofactor specificity.

### 2.1 Methods

### 2.1.1 Aminoacid residue sequences and protein structure templates

The wild-type amino acid residue sequences from the selected case-study enzymes were retrieved from Uniprot, while mutant sequences were replicated in silico according to the specifications presented in the literature. Uniprot IDs from the four selected enzymes are as following: Pichia stipitis's xylose reductase: P31867; Gluconobacter oxydans's xylitol dehydrogenase: Q8GR61; Pichia stipitis's xylitol dehydrogenase: P22144; Tramitichromis intermedius's leucine dehydrogenase: Q60030.

Gluconobacter oxydans' xylitol dehydrogenase had its structure already experimentally characterized (PDB id: 1ZEM), being therefore used as a template for modeling the structure of the predicted mutants. As for the remaining three enzymes, since their structures were not experimentally characterized, the structures of the wild-type and corresponding mutants were generated using comparative modeling. Pichia stipitis' xylose reductase structure was modeled using the homologue structure of Arabidopsis thaliana's aldo-keto reductase (PDB id: 3H7R) with 47% identity; Pichia stipitis' xylitol dehydrogenase structure was modeled using the homologue structure of Homo sapiens' sorbitol dehydrogenase (PDB id: 1PL6) with 44% identity and Tramitichromis intermedius' leucine dehydrogenase structure was modeled using the homologue structure of Rhodococcus sp. M4's phenylalanine dehydrogenase (PDB id: 1BW9) with 37% identity.

### 2.1.2 Cofactor specificity prediction

Predictions on cofactor specificity change were performed using NiCofactor, the method described previously in the description, for allowing the high throughput NAD(P)(H) cofactor specificity prediction. NiCofactor was built using the python programming language. Input sequences are required to be in FASTA format. For each sequence, the tool initiates an individual project. The tools for generating CNRPMs and performing machine learning were also integrated in NiCofactor. Results are outputted by attributing to each analyzed sequence a cofactor prediction and subsequent prediction score. The default probability score threshold used is 0.8.

### 2.1.3 Evolutionary algorithm implementation

The evolutionary algorithms used in the stochastic method for efficiently predict the optimal set of mutations to reverse cofactor specificity were implemented using inspyred [16], an open source framework for creating biologically-inspired computational intelligence algorithms in Python.

Five evolutionary algorithms were implemented, with the only difference between them being the maximum candidate size allowed. Each algorithm was configured to run through 100 generations, being the initial population composed by 100 individuals. Each individual was randomly created according to the candidate maximum size, which varied between 1 and 5. This corresponds to the creation of mutant amino acid residue sequences derived from the original target aminoacid residue sequence, containing between 1 and 5 mutations each. Elitism value was set to 2, keeping the best 2 scoring individuals for the next generation. The next best scoring 50 individuals were recombined using mutation operators, with a crossover rate of 0.9 and a mutation rate of 0.1. The crossover operator uses the parameters of two individuals and combines them, generating two new individuals, while the mutation operator substitutes one element of the individual by another, randomly generated. The remaining lowest scoring 48 individuals were discarded and newly generated individuals with random mutations in the available mutable positions were incorporated in the population. The optimization process is terminated when the maximum number of generations is achieved.

### 2.1.4 Protein structure visualization

Wild-type and mutant enzyme structures were visualized using PyMol [17] a free and user-friendly molecular graphics system for molecular visualization written in Python programming language.

### 2.2 Results and discussion

### 2.2.1 Identification of mutable residue positions for cofactor specificity reversal

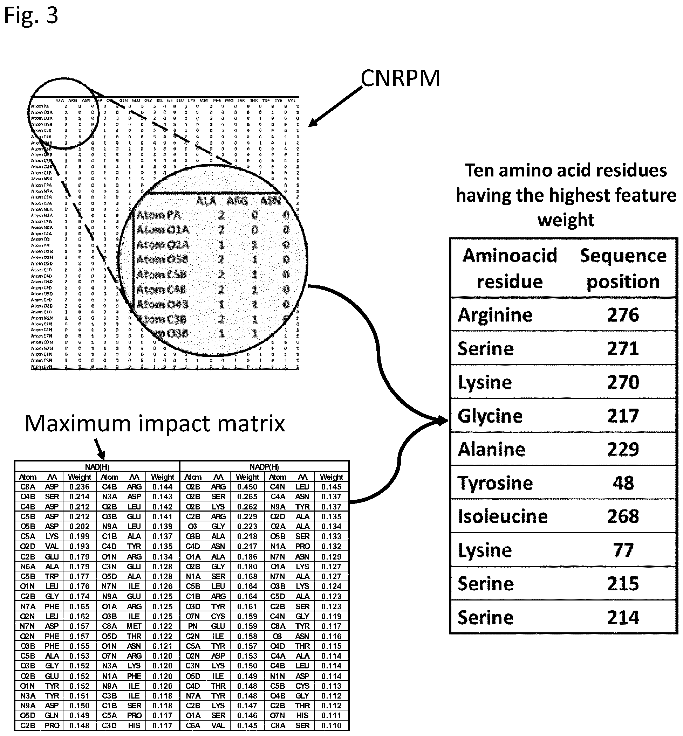

As previously stated, the SVM model, trained with a large dataset of CNRPMs, performed the attribution of importance scores to the features in the dataset, allowing the correct separation of the instances in the hyperplane. In this case, the features are composed of relations between the atoms in each cofactor and the corresponding neighbor amino acid residues. Then, the score, i.e. the feature weight, attributed to each feature, reveals the impact of each interaction in the binding preference of the cofactors, with higher scoring features having a higher impact on cofactor specificity. Figure 3 depicts a representation of the process undertaken for the selection of mutable residue positions for cofactor specificity reversal. Through the analysis of the CNRPM generated from a target enzyme's structure, and by combining this information with the data stored in the SVM model, the sorting of the best features for cofactor specificity is performed. When the most influential features for cofactor specificity in a given protein structure are sorted, the corresponding residue position in the sequence is retrieved and stored for each feature. When features from ten distinct residues in the sequence are retrieved, the list is closed.

The end result is an ordered list of the most influential residues in cofactor specificity for a given target enzyme, being assumed that, due to their influence in cofactor specificity, this list contains the optimal mutable residues for cofactor specificity reversal.

Despite the developed method's utility in precisely pinpointing suitable target residues for cofactor specificity reversal mutations, the computational costs or comparably high due to the combinatory amount of total mutant possibilities. In order to optimize this task, two distinct approaches were undertaken, resulting in the development of two different methods for determining the optimal set of mutations necessary to achieve cofactor specificity reversal.

### 2.2.2 Cofactor specificity reversal - Deterministic method

With the intent of surpassing the overwhelming amount of combinatorial mutations necessary for the screening of all suitable mutable residue positions to achieve the optimal cofactor specificity reversal mutant, a deterministic method was developed. This deterministic approach is based on the formulation of a hypothesis regarding both cofactor's most influential features. With this in mind, the suggested hypothesis states that: if, for each atom composing a cofactor, there is a specific neighbor amino acid residue with the highest impact in the cofactor specificity, then, if this interacting amino acid residue is replaced by the amino acid residue with the highest impact for the opposite cofactor, the binding specificity should be affected. Therefore, if enough amino acid residues with high impact on cofactor specificity are changed, the cofactor specificity should be reverted.

In order to implement the stated hypothesis, the extracted SVM feature weights present in appendix, in table A1, were further examined. For each selected cofactor atom, the strongest feature present for NAD(H) and NADP(H) specificity was selected, and the associated amino acid residue retrieved. The resulting chart, displayed in table 2.1, represents, for each selected cofactor atom, the amino acid residue interaction with highest impact on cofactor specificity, and consequently the candidate for performing point-mutations in that area of the binding spot.

**Table 2.1. Point-mutation selecting chart. Each selected cofactor atom and corresponding molecular localization is represented in the column "cofactor atom". For each atom, the corresponding NAD(H) and NADP(H) specificity is represented in columns "NAD(H) specific" and "NADP(H) specific" respectively. This table corresponds to the impact matrix.**

| **Cofactor atom** | **NAD(H) specific** | **NADP(H) specific** | **Cofactor atom** | **NAD(H) specific** | **NADP(H) specific** |
|---|---|---|---|---|---|
| **PA** | **GLY** | **GLU** | **O3** | **HIS** | **GLY** |
| **O1A** | **ARG** | **ALA** | **PN** | **ASN** | **GLU** |
| **O2A** | **GLU** | **ALA** | **O1N** | **LEU** | **TRP** |
| **O5B** | **ASP** | **SER** | **O2N** | **LEU** | **ASP** |
| **C5B** | **ASP** | **LEU** | **O5D** | **GLN** | **ILE** |
| **C4B** | **ASP** | **LEU** | **C5D** | **SER** | **ALA** |
| **O4B** | **SER** | **GLY** | **C4D** | **TYR** | **ASN** |
| **C3B** | **ILE** | **CYS** | **O4D** | **ILE** | **THR** |
| **O3B** | **PHE** | **ALA** | **C3D** | **HIS** | **TYR** |
| **C2B** | **GLU** | **ARG** | **O3D** | **THR** | **TYR** |
| **O2B** | **GLU** | **ARG** | **C2D** | **PRO** | **GLU** |
| **C1B** | **ALA** | **ARG** | **O2D** | **VAL** | **ALA** |
| **N9A** | **ASP** | **TYR** | **C1D** | **CYS** | **THR** |
| **C8A** | **ASP** | **TYR** | **N1N** | **PRO** | **ASP** |
| **N7A** | **PHE** | **TYR** | **C2N** | **MET** | **ILE** |
| **C5A** | **LYS** | **TYR** | **C3N** | **GLU** | **LYS** |
| **C6A** | **ALA** | **VAL** | **C7N** | **ASN** | **GLN** |
| **N6A** | **ALA** | **GLN** | **O7N** | **ARG** | **CYS** |
| **N1A** | **PHE** | **SER** | **N7N** | **ASP** | **ASN** |
| **C2A** | **ASP** | **ALA** | **C4N** | **ALA** | **LEU** |
| **N3A** | **TYR** | **ARG** | **C5N** | **ASN** | **ILE** |
| **C4A** | **PHE** | **ASN** | **C6N** | **GLU** | **PHE** |

### 2.2.2.1 Implementation of the deterministic method for cofactor specificity conversion

The implementation of the deterministic method for the conversion of NAD(P)(H) cofactor specificity, starts with the structural analysis of the subject enzyme and respectively CNRPM assembly with NiCofactor. If the subject enzyme's structure is not characterized, homology modelling with a suitable structural template is performed automatically by NiCofactor.

Once created, CNRPM features are sorted and the one with the highest impact is selected. The amino acid residue sequence position from the residue present in the feature is retrieved, while the atom present in the feature is searched in the point-mutation selecting chart displayed in table 2.1, being the candidate amino acid residue mutant selected.

The wild-type amino acid residue is replaced by the mutant candidate in the amino acid residue sequence. The mutant sequence is retrieved and its cofactor specificity is predicted using NiCofactor.

If a prediction is performed, with a probability score above the threshold value, and the cofactor predicted for the mutant sequence is changed, the mutant sequence is accepted as having its cofactor specificity successfully altered. If, on the other hand, the cofactor prediction did not change, the conversion method continues with the mutant sequence and the second highest impact feature is used. This step is performed iteratively, with mutations being incremented in the sequence until the cofactor prediction is changed. If after 10 consecutive mutations the cofactor prediction remains unaltered, the mutation is regarded as unviable.

For example, for the NAD(H) dependent target enzyme structure, the atom-amino acid residue interaction with highest impact on cofactor specificity is originated by the presence of a Phenylalanine (F) near the atom O3B, in the ribose from the adenine moiety. By consulting table 2.1 it is possible to observe that the residue originating the atom-amino acid residue interaction, with atom O3B, with the highest impact on cofactor specificity for NADP(H) is an Alanine (A). Being the sequence position of the selected Phenylalanine, position 42, a point mutation is performed and the Phenylalanine is substituted by an Alanine. Despite this mutation, the cofactor specificity prediction of the target enzyme was not altered, being therefore selected the second highest impact interaction, the Aspartate (D) near the atom O5B. With the substitution of Aspartate by a Serine (S) not rendering an altered cofactor specificity prediction, the third highest impact interaction was selected. This time, the Aspartate near atom C8A was mutated into a Tyrosine (Y) and the resulting mutant F42A/D23S/D71Y was successfully predicted as having reverted its original cofactor specificity.

The presented deterministic method is a fast and precise approach for the complex problem of selecting the optimal set of mutations capable of reverting cofactor specificity in a target enzyme. Despite its overall efficacy, robustness and time efficiency, the deterministic characteristics of this approach mean that there are multiple mutation combinations that are not taken into consideration, with the possibility of better results for a set of cofactor specificity reverting mutations being overlooked. Due to these constraints, and in order to analyze the highest number of mutation combinations possible, a stochastic method was developed, with the incorporation of an evolutionary algorithm.

### 2.2.3 Cofactor specificity reversal - Stochastic method

With the selection of the ten most suitable mutable residue positions for cofactor specificity reversal, and given the possibility of each residue position being mutated by the remaining 19 amin oacids residues, it becomes clear the impossibility of predicting the cofactor specificity of every mutant combination. To overcome this issue, a stochastic method was developed through the implementation of an evolutionary algorithm. These optimization algorithms perform the evolution of a population by mimicking biologic events such as natural selection. Each individual in a population is evaluated through a fitness function and compared with newly generated individuals created by the application of reproduction operators to selected parents. As in nature, only the fittest individuals are allowed to continue in the population and reproduce [18].

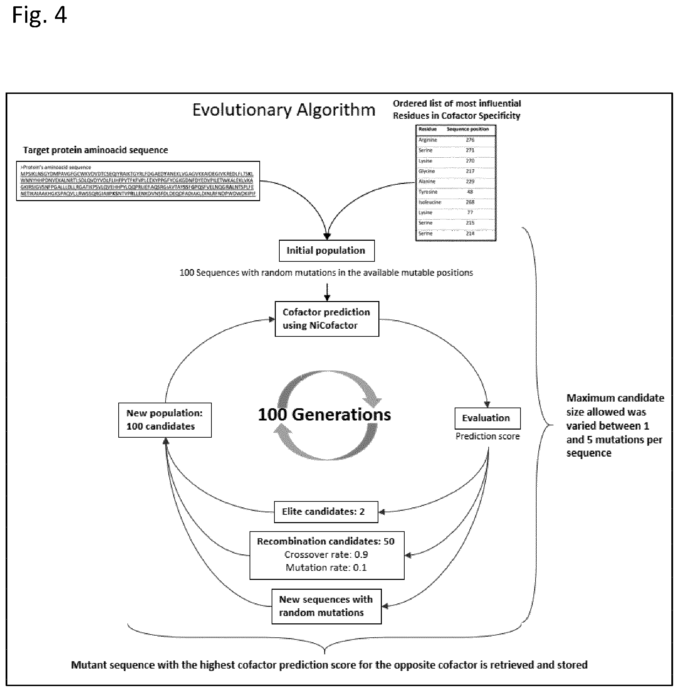

Figure 4 is depicts a representation of the implemented evolutionary algorithms. Given the target amino acid residue sequence and the list of 10 mutable positions, an initial population of 100 mutant amino acid residue sequences (individuals) was generated, with random mutations in the available mutable positions. In this work, 5 evolutionary algorithms were implemented, with the only difference being the maximum candidate size allowed, varying between 1 and 5 mutations per individual. The optimization process was run for 100 generations. During each generation, the cofactor specificity of each individual was predicted using NiCofactor. After evaluating the entire population, the two best scoring individuals were maintained for the next generation, while the next best 50 undertook a recombination process, being 90% by crossover, where two individuals are crossed over to generate two new individuals, and 10% by mutation, where an individual's suitable aminoacid residue is randomly mutated. The remaining lowest scoring 48 individuals were discarded and newly generated individuals with random mutations in the available mutable positions were incorporated in the population. In the end of the optimization process, the five mutant sequences, containing 1 to 5 mutations, with the highest cofactor prediction score for the opposite cofactor were retrieved and outputted as result.

### 2.2.4 Case studies

With the intent of assessing the performance of the developed methods for cofactor specificity reversal, four case studies were replicated in silico and their cofactor specificity reverted, using the developed methods. From the group of cofactor engineering studies published by Khoury and coworkers [19], the four enzymes that were found to have completely reverted specificity or largely decreased affinity for one of the cofactors, increasing the affinity of the other, were selected as case studies. These were the cases of xylose reductase from Picchia stipitis (PsXR) [20], xylitol dehydrogenase from Gluconobacter oxydans (GoXD) [21], xylitol dehydrogenase from Pichia stipitis (PsXD) [22] and leucine dehydrogenase from Tramitichromis intermedius (TiLD) [23]. For these enzymes, NiCofactor was able to correctly predict the cofactor specificity of both wild-type and specificity reversed mutants. With that in mind, the four enzymes' amino acid residue sequence were retrieved and processed using the above described methods with the intent of showcasing the results achieved in silico, and comparing them to the experimentally determined data on cofactor specificity reversing mutations.

From the four enzymes analyzed, three are part of the xylose metabolism, an extremely important pathway due to its great economical potential. Being a major component of hemicellulose and only second to glucose as the most abundant sugar in nature, D-xylose can be bioconverted from agricultural biomass wastes into biofuels, such as ethanol, through fermentation processes. However, Saccharomyces cerevisiae, the best adapted microorganism for producing ethanol, is not genetically equipped for metabolizing xylose. To solve this problem, xylose fermenting genes have been cloned in S. cereviseae from other organisms capable of metabolizing this sugar, such as Pichia spitipis [20], [22] and Gluconobacter oxidans [21]. Xylose reductase (EC 1.1.1.21) reduces xylose into xylitol using NADPH and xylitol dehydrogenase (EC 1.1.1.9) oxidizes, posteriorly, xylitol into xylulose, using NAD+. Nonetheless, this difference in cofactor specificity creates an intercellular redox unbalance, hindering ethanol production yields and promoting xylitol excretion. An elegant solution implemented to solve this problem is the cofactor specificity reversal of xylose reductase from NADPH to NADH [20] or, by alternative, the specificity reversal of xylitol dehydrogenase from NAD+ to NADP+, taking advantage of the often higher availability of NADP+ in the cell [21], [22].

The remaining enzyme, Leucine dehydrogenase from Thermoactinomyces intermedius uses NAD+ for catalyzing the reversible deamination of L-leucine to its 2-oxo analogue, 4-methyl-2-oxopentanoate. As biosynthesis reactions generally use NADP+ as cofactor, leucine dehydrogenase cofactor specificity reversal might improve this reaction's efficiency [23].

### 2.2.4.1 PsXR - Pichia stipitis xylose reductase

Xylose reductase (PsXR), from Pichia stipitis, was the only enzyme in the analyzed group with cofactor specificity for NADP(H), with the remaining enzymes being specific for NAD(H). In table 5.2 the results achieved for the in silico cofactor specificity change of PsXR are displayed.

As previously stated, NiCofactor was able to correctly predict the cofactor specificity from both wild-type and literature cofactor reversed mutant, being the results achieved by the deterministic and stochastic methods only outputted when the prediction score threshold is achieved.

**Table 2.2. Mutations, cofactor predictions and prediction scores from literature experimental data, as well as from the implementation of both methods for reversing cofactor specificity in silico of PsXR. The deterministic method outputs only one mutant, while the stochastic method outputs five different mutants with the best found set of mutations for specificity reversal, according to the maximum candidate size allowed by the method, with the number on the gene name corresponding to the number of mutations selected.**

| **Gene name** | **Mutation** | **Predicted cofactor** | **Prediction score** |
|---|---|---|---|
| **PsXR** | Wild-type | NADP | 0.8764 |
| **PsXR Literature** | K270S/S271G/N272P/R276F | NAD | 0.8792 |
| **PsXR Deterministic** | S271E/R276E | NAD | 0.9747 |
| **PsXR Stochastic 1** | R276D | NAD | 0.8661 |
| **PsXR Stochastic 2** | K270D/S271D | NAD | 0.9773 |
| **PsXR Stochastic 3** | K270D/S271D/R276D | NAD | 0.9996 |
| **PsXR Stochastic 4** | S215R/K270D/S271D/R276D | NAD | 0.9995 |
| **PsXR Stochastic 5** | G217D/I268R / K270D/S271D/R276D | NAD | 0.9999 |

When analyzing table 2.2 it is also possible to observe the amount and type of point mutations recommended by the developed methods in order to achieve cofactor specificity reversal. Being the literature mutant composed by four point-mutations, achieved through the implementation of a combinatorial active-site saturation mutagenesis method, we can observe that both deterministic and stochastic methods here implemented were able to predict mutants with fewer point-mutations and higher predicted reversed cofactor specificity. In this case, PsXR Deterministic is composed by only two point-mutations, with an Arginine (R) and a Serine (S) being substituted by a Glutamate (E). In the case of PsXR stochastic, the evolutionary algorithm was able to find a mutant with predicted reversed cofactor specificity with only one point-mutation, being this individual, due to its lower amount of point-mutations, considered the best hypothesis for performing in vivo cofactor specificity reversal. When analyzing the remaining stochastic mutants, it is observed that the mutants with higher amounts of point-mutations tend to incorporate the point-mutations predicted for the stochastic mutants with fewer point-mutations, indicating a strong effect of these mutations for the reversal of cofactor specificity. We can also see, in the stochastic mutants, that the cofactor prediction scores increase with the amount of point-mutations predicted, however, preference should be given to mutants with fewer mutations in order to preserve the structural stability of the enzyme.

### 2.2.4.2 GoXD -Gluconobacter oxydans xylitol dehydrogenase

Xylitol dehydrogenase (GoXD), from Gluconobacter oxydans, oxidizes xylitol into xylulose and has been shown to use exclusively NAD+ as a reaction cofactor [21]. Table 5.3 displays the results achieved for the in silico cofactor specificity change of GoXD from NAD+ dependent to NADP+.

**Table 2.3. lists mutations, cofactor predictions and prediction scores from literature experimental data, as well as from the implementation of both methods for reversing cofactor specificity in silico of GoXD. The deterministic method outputs only one mutant, while the stochastic method outputs five different mutants with the best found set of mutations for specificity reversal, according to the maximum candidate size allowed by the method, with the number on the gene name corresponding to the number of mutations selected.**

| **Gene name** | **Mutation** | **Predicted cofactor** | **Prediction score** |
|---|---|---|---|
| **GoXD** | Wild-type | NAD | 0.9678 |
| **GoXD Literature** | D38S/M39R | NADP | 0.9541 |
| **GoXD Deterministic** | D38Y/A92Q/G93R | NADP | 0.8822 |
| **GoXD Stochastic 1** | D38R | NADP | 0.8668 |
| **GoXD Stochastic 2** | D38R/A92R | NADP | 0.9752 |
| **GoXD Stochastic 3** | D38R/D64R/A92R | NADP | 0.9944 |
| **GoXD Stochastic 4** | G16K/D38R/D64S/G93R | NADP | 0.9957 |
| **GoXD Stochastic 5** | G14S/G16T/D38R/D64R/A92R | NADP | 0.9999 |

The results displayed in table 2.3 show a high score for the prediction of cofactor specificity of wild-type and literature experimental data, increasing the confidence level on the agreement between predicted and experimental results. In the analyzed case-study, the literature mutant was achieved with only two point-mutations, while the deterministic method required three mutations to achieve the same cofactor specificity prediction. As to GoXD stochastic results, the developed method was able to output a predicted reversed cofactor specificity mutant encompassing only one point-mutation, being it considered the best hypothesis for performing in vivo cofactor specificity reversal with minimal interventions. When further analyzing the achieved results, it is possible to observe that both the literature mutant and the selected stochastic mutant share similar features despite being originated from different approaches. From these approaches, the one described in the literature is the most laborious, involving structure characterization and structural alignment with other enzymes, together with the multiple selections of conserved amino acid residue positions. In the literature mutant an Aspartate residue in position 38 was deleted and an Arginine residue was incorporated in position 39, whereas in the stochastic mutant, this event occurred in the same spot, position 38. A common characteristic that the predicted mutations appear to possess, in order to successfully reverting cofactor specificity in this case, is the promotion of Arginine (R) inclusion and the exclusion of Aspartate (D) in the analyzed sequences.

### References

[1] S. Placzek, I. Schomburg, A. Chang, L. Jeske, M. Ulbrich, J. Tillack, and D. Schomburg, "BRENDA in 2017: New perspectives and new tools in BRENDA," Nucleic Acids Res., vol. 45, no. D1, pp. D380-D388, 2017.
[2] T. Hamelryck and B. Manderick, "PDB file parser and structure class implemented in Python," Bioinformatics, vol. 19, no. 17, pp. 2308-2310, 2003.
[3] F. Pedregosa, G. Varoquaux, A. Gramfort, V. Michel, B. Thirion, O. Grisel, M. Blondel, P. Prettenhofer, R. Weiss, V. Dubourg, J. Vanderplas, A. Passos, D. Cournapeau, M. Brucher, M. Perrot, and E. Duchesnay, "Scikit-learn: Machine Learning in Python," J. Mach. Learn. Res., vol. 12, pp. 2825-2830, 2012.
[4] S. Van Der Walt, S. Africa, and M. S. Feb, "The NumPy array: a structure for efficient numerical computation," pp. 1-8, 2011.
[5] B. W. Matthews, "COMPARISON OF THE PREDICTED AND OBSERVED SECONDARY STRUCTURE OF T4 PHAGE LYSOZYME," Biochim. Biophys. Acta, vol. 405, pp. 442-451, 1975.
[6] J. Swets, "Measuring the accuracy of diagnostic systems," Science (80-. )., vol. 240, no. 4857, pp. 1285-1293, 1988.
[7] B. Webb and A. Sali, Comparative protein structure modeling using MODELLER, vol. 2014. 2014.
[8] T. Smith and M. Waterman, "Identification of common molecular subsequences," J. Mol. Biol., vol. 147, no. 3, pp. 195-197, 1981.
[9] W. R. Pearson, "Empirical Statistical Estimates for Sequence Similarity Searches," J. Mol. Biol., vol. 276, pp. 71-84, 1998.
[10] D. Box, D. Ehnebuske, G. Kakivaya, A. Layman, N. Mendelsohn, H. Nielsen, S. Thatte, and D. Winer, "Simple Object Access Protocol (SOAP) 1 . 1," W3C - World Wide Web Consort. Note, no. October, 2000.
[11] C. Cortes and V. Vapnik, "Support Vector Networks," Mach. Learn., vol. 20, no. 3, pp. 273-297, 1995.
[12] S. Watanabe, M. Yamada, I. Ohtsu, and K. Makino, "a-Ketoglutaric semialdehyde dehydrogenase isozymes involved in metabolic pathways of D-glucarate, D-galactarate, and hydroxy-L-proline: Molecular and metabolic convergent evolution," J. Biol. Chem., vol. 282, no. 9, pp. 6685-6695, 2007.
[13] X. Liu, Y. Dong, J. Zhang, A. Zhang, L. Wang, and L. Feng, "Two novel metal-independent long-chain alkyl alcohol dehydrogenases from Geobacillus thermodenitrificans NG80-2," Microbiology, vol. 155, pp. 2078-2085, 2009.
[14] B. Ahvazi, R. Coulombe, M. Delarge, M. Vedadi, L. Zhang, E. Meighen, and A. Vrielink, "Crystal structure of the NADP+-depe,indent aldehyde dehydrogenase from Vibrio harveyi: structural implications for cofactor specificity and affinity.," Biochem. J., vol. 349 Pt 3, pp. 853-61, 2000.
[15] O. Carugo and P. Argos, "NADP-dependent enzymes. I: Conserved stereochemistry of cofactor binding," Proteins Struct. Funct. Genet., vol. 28, no. 1, pp. 10-20, 1997.
[16] J. Barhak and A. Garrett, "Population Generation from Statistics Using Genetic Algorithms with MIST + INSPYRED," MODSIM World 2014, pp. 1-8, 2014.
[17] W. DeLano, "Pymol: An open-source molecular graphics tool," CCP4 Newsl. Protein Crystallogr., vol. 700, 2002.
[18] Z. Michalwicz, "Evolutionary Programming and Genetic Programming," Genet. Algorithms + Data Struct. = Evol. Programs, vol. 13, pp. 283-287, 1996.
[19] G. A. Khoury, H. Fazelinia, J. W. Chin, R. J. Pantazes, P. C. Cirino, and C. D. Maranas, "Computational design of Candida boidinii xylose reductase for altered cofactor specificity," Protein Sci., vol. 18, no. 10, pp. 2125-2138, 2009.
[20] L. Liang, J. Zhang, and Z. Lin, "Altering coenzyme specificity of Pichia stipitis xylose reductase by the semi-rational approach CASTing," Microb. Cell Fact., vol. 6, pp. 1-11, 2007.
[21] A. H. Ehrensberger, R. A. Elling, and D. K. Wilson, "Structure-guided engineering of xylitol dehydrogenase cosubstrate specificity," Structure, vol. 14, no. 3, pp. 567-575, 2006.
[22] S. Watanabe, T. Kodaki, and K. Makino, "Complete reversal of coenzyme specificity of xylitol dehydrogenase and increase of thermostability by the introduction of structural zinc," J. Biol. Chem., vol. 280, no. 11, pp. 10340-10349, 2005.
[23] a Galkin, L. Kulakova, T. Ohshima, N. Esaki, and K. Soda, "Construction of a new leucine dehydrogenase with preferred specificity for NADP+ by site-directed mutagenesis of the strictly NAD+-specific enzyme.," Protein Eng., vol. 10, no. 6, pp. 687-690, 1997.

## Claims

1. A method for determining a cofactor specificity of a target enzyme, wherein the target enzyme is expected to use one of a first cofactor and a second cofactor based on an amino-acid sequence of the target enzyme, and/or for determining an amino-acid sequence of a target enzyme variant, wherein the variant is **characterized by** a cofactor specificity differing from that of the target enzyme, the method comprising at least the steps of:
i) providing an atomic structure for each of both cofactors, wherein each atomic structure comprises cofactor atoms, and wherein cofactor atoms in the atomic structures that are located at the same corresponding locations in both atomic structures are selected,
ii) providing the amino-acid sequence of the target enzyme,
iii) determining an estimated target enzyme-cofactor structure comprising information on a spatial structure of the target enzyme bound to one of the cofactors,
iv) generating an interaction matrix for the target enzyme-cofactor structure, wherein the interaction matrix comprises entries relating the selected cofactor atoms to surrounding amino-acid residues of the target enzyme, particularly wherein for each selected cofactor atom, entries are generated in the interaction matrix that comprise the counts of each amino-acid residue of the target enzyme within a predefined distance to the selected cofactor atom,
v) determining a cofactor specificity of the target enzyme by providing the interaction matrix, particularly the entries of the interaction matrix, to a trained classifier that is configured to classify the cofactor specificity of the target enzyme based on the provided interaction matrix to either the first or the second cofactor.

2. The method according to claim 1, wherein particularly if the target enzyme-cofactor structure is unknown, the following steps are executed for determining the estimated target enzyme-cofactor structure:
- performing a homology search with the amino-acid sequence of the target enzyme in a protein structure database comprising information on molecular structures of enzymes bound to the first or the second cofactor,
- assigning the molecular structure comprising the enzyme that exhibits a highest degree of homology to the amino-acid sequence of the target enzyme as a molecular structure template,
- from the molecular structure template determining the target enzyme-cofactor structure for the amino-acid sequence of the target enzyme bound to the cofactor particularly by aligning the amino-acid sequence of the target enzyme to the molecular structure template.

3. The method according to any one of the preceding claims, wherein the classifier is trained by
- providing information on molecular structures of a plurality of enzymes, wherein each molecular structure represents an enzyme bound to the first or the second cofactor,
- for each molecular structure, generating the interaction matrix, wherein each interaction matrix is associated to the respective cofactor of the molecular structure,
- training the classifier with the interaction matrices so that the classifier is configured to classify the cofactor specificity for the first or the second cofactor of an enzyme based on the entries of the interaction matrix.

4. The method according to any one of the preceding claims, wherein the trained classifier provides a cofactor specificity probability for the first and/or the second cofactor for the interaction matrix for classifying the cofactor specificity of the target enzyme, wherein if said probability exceeds a predefined threshold value, the target enzyme is classified to be specific to the respective cofactor.

5. The method according to any one of the preceding claims, wherein the amino-acid sequence of the target enzyme having the cofactor specificity switched from the first cofactor to the second cofactor is determined by the steps of:
a) particularly providing the target cofactor enzyme structure with a target enzyme being specific to the first cofactor,
b) prior to step v), replacing at least one amino-acid in the interaction matrix,
c) determining whether the cofactor specificity determined in step v) is switched from the first cofactor to the second cofactor,
d) particularly repeating the steps b) to c), particularly until the cofactor specificity determined in step v) is switched from the first cofactor to the second cofactor.

6. The method according to any one of the preceding claims, wherein for each of the two cofactors and for each of the selected cofactor atoms and for each amino-acid, a feature weight indicative of a cofactor specificity strength is determined or provided, particularly wherein the trained classifier determines and/or provides the feature weights after being trained, particularly wherein said feature weights are provided in form of a computer readable look-up table.

7. The method according to claim 6, wherein a maximum impact matrix is determined or provided relating each selected cofactor atom to the amino-acid having the largest feature weight for the cofactor specificity for the first cofactor and the second cofactor, particularly wherein the maximum impact matrix is determined and/or provided by the trained classifier, particularly wherein the maximum impact matrix stored as a computer readable look-up table.

8. The method according to any one of the preceding claims, wherein the amino-acid sequence of the target enzyme having the cofactor specificity switched from the first cofactor to the second cofactor is determined by the steps of:
- before the interaction matrix is provided to the trained classifier in step v), replacing M amino-acid residues in the interaction matrix corresponding to the amino-acid residues of the target enzyme with the M largest feature weights for the first cofactor, with the corresponding M amino-acid residues for the same cofactor atoms having the largest feature weight for the second cofactor, wherein M is a natural number,
- particularly providing the such altered interaction matrix to the trained classifier as the interaction matrix in step v),
- after step v), determining whether the specificity of the enzyme variant associated to the interaction matrix is switched with respect to the target enzyme.

9. The method according to claim 8, wherein starting from an initial value for M, particularly M =1, the steps of claim 8 are repeated, wherein M is incremented by one or more during each repetition, until the cofactor specificity of the target enzyme is switched or a predefined maximum value for M is reached.

10. The method according to any one of the claims 5 to 9, wherein the amino-acid sequence of the target enzyme having the cofactor specificity switched from the first cofactor to the second cofactor is determined by the steps of:
I) before the interaction matrix is provided to the trained classifier in step v), determining N amino-acid residues of the target enzyme with the N largest feature weights for the first cofactor, wherein N is a natural number, particularly 10,
II) repeatedly performing a stochastic evolutionary method for replacing the N amino-acid residues with other amino-acid residues in the interaction matrix, and for each cycle of the stochastic evolutionary method and for each amino-acid sequence retrieved from the cycle determining the cofactor specificity and the cofactor specificity probability with step v) with the trained classifier,
III) after step v) of claim 1, selecting at least one target enzyme with a switched cofactor specificity.

11. The method according to claim 10, wherein in step II) selected amino sequences are submitted to a next cycle of the stochastic evolutionary method, wherein the amino-acid sequences are selected based on the highest cofactor probability for the second cofactor.

12. The method according to one of the preceding claims, wherein the first cofactor is selected from one of the redox pairs NAD / NADH and NADP / NADPH, and the second cofactor is the other of the redox pairs.

13. Method according to one of the preceding claims, wherein the target enzyme is synthesized.

14. A computer program comprising instructions which, when the program is executed by a computer, cause the computer to carry out the method according to any of the claims 1 to 12.
